# EUROPEAN PATENT APPLICATION

(11) **EP 3 434 666 A1**
(43) Date of publication of application: **30.01.2019**
(21) Application number: 17770200.8
(22) Date of filing: 21.03.2017
(51) Int. Cl.: C07C 211/54, C07C 209/10, C07D 209/88, C09K 11/06, H01L 51/50, H05B 33/10, C07B 61/00

(54) **ARYLAMINE DERIVATIVE AND USE THEREOF**

(30) Priority: 24.03.2016 JP 2016060215; 22.06.2016 JP 2016123323
(71) Applicant: Nissan Chemical Corporation, Tokyo 103-6119 (JP)
(72) Inventor: OTANI, Naoki, Funabashi-shi Chiba 274-0052 (JP); TERAI, Seiya, Funabashi-shi Chiba 274-0052 (JP)
(74) Representative: Mewburn Ellis LLP
(86) International application number: PCT/JP2017/011169
(87) International publication number: WO 2017/164158

(57) **Abstract**

This arylamine derivative represented by formula (1) exhibits good solubility to an organic solvent, and can be used to provide a thin film having solvent resistance, and also provides an organic EL element which exhibits good characteristics when the arylamine derivative is applied to a positive hole transport layer. 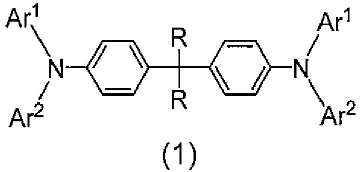 (In the formula, each R independently represents a fluorine atom-containing alkyl group having 1-5 carbon atoms, each Ar¹ independently represents an aryl group that has 6-20 carbon atoms, that is optionally substituted with Z¹, and that has a crosslinking group, each Ar² independently represents at least one aryl group selected from those represented by formulae (2)-(4), and Z¹ represents a halogen atom, a nitro group, a cyano group, an amino group, or an alkyl group having 1-20 carbon atoms optionally substituted with Z⁴.) 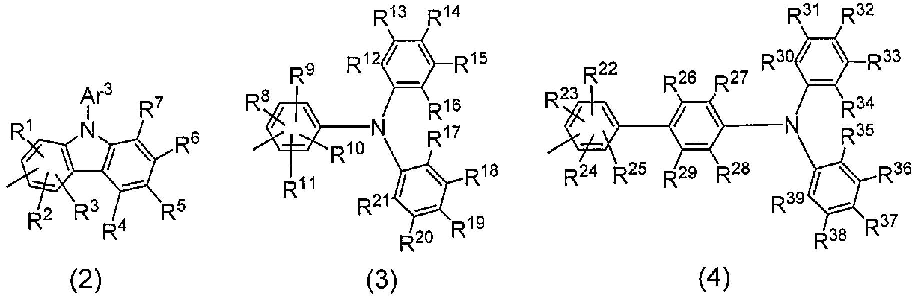 (In the formula, Ar³ represents a hydrogen atom, or an alkyl group having 6-20 carbon atoms optionally substituted with Z², and R¹-R³⁹ each independently represent a hydrogen atom, a halogen atom, etc.)

## Description

### TECHNICAL FIELD

The present invention relates to an arylamine derivative and use thereof.

### BACKGROUND ART

For an organic electroluminescence (hereinafter referred to as organic EL) element, a charge-transporting thin film made of an organic compound is used as a light-emitting layer and a charge injection layer. In particular, a hole injection layer causes charges to be exchanged between an anode and a hole-transporting layer or a light-emitting layer, and plays an important role in order to achieve the high luminance and the low-voltage driving of the organic EL element.

Methods of forming the hole injection layer are broadly divided into a dry process such as vapor deposition and a wet process such as spin coating. The comparison of these processes indicates that the wet process enables the efficient manufacture of a thin film with higher flatness in a larger area. Therefore, in the current situation that the organic EL display is enlarged, the hole injection layer that can be formed by the wet process has been expected.

In view of the above circumstances, the present inventors have developed a charge-transporting material that is applicable to various wet processes and that can form a thin film for achieving the excellent EL element characteristic when the thin film is used for the hole injection layer of the organic EL element, and a compound that is highly soluble to an organic solvent used for the charge-transporting material (for example, see Patent Documents 1 to 4).

In recent years, however, it has been demanded that not just the hole-transporting layer and the hole injection layer but also the light-emitting layer and the like are manufactured by the wet process. In this case, the hole-transporting layer and the hole-injecting-and-transporting layer to be the base are required to have the resistance against the solvent used in the composition for forming the light-emitting layer. In the materials according to Patent Documents 1 to 4, however, there is room for improvement in this perspective.

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777

### SUMMARY OF INVENTION

### TECHNICAL PROBLEM

An object of the invention, which has been made under the above circumstances, is to provide an arylamine derivative for forming a thin film that is highly soluble to an organic solvent and that has solvent resistance, and for forming an organic EL element with excellent characteristics when used as the hole-transporting layer.

### SOLUTION TO PROBLEM

Making extensive investigations to attain the above object, the inventors have found that: a specific arylamine derivative with an aryl group containing a cross-linking group is highly soluble to an organic solvent and by heating a varnish obtained by dissolving the arylamine derivative into the organic solvent and thermally cross-linking the arylamine derivative, a thin film with the excellent solvent resistance can be obtained; and in a case where the thin film is used for a hole-transporting layer of an organic EL element, the organic EL element can have excellent luminous efficiency. Thus, the present invention has been attained.

That is to say, the present invention provides:
1. An arylamine derivative represented by formula (1): 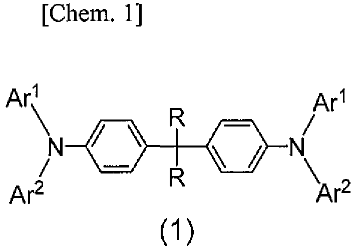 [wherein, R independently represents an alkyl group with 1 to 5 carbon atoms containing a fluorine atom, Ar¹ independently represents an aryl group with 6 to 20 carbon atoms that includes a cross-linking group and may be substituted by Z¹, Ar² independently represents at least one aryl group selected from formulae (2) to (4), and Z¹ represents a halogen atom, a nitro group, a cyano group, an amino group, or an alkyl group with 1 to 20 carbon atoms that may be substituted by Z⁴, 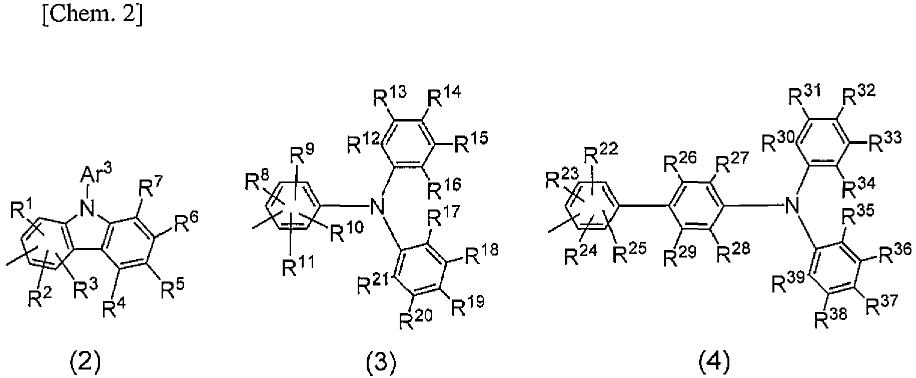
   (wherein, Ar³ represents a hydrogen atom or an aryl group with 6 to 20 carbon atoms that may be substituted by Z²;
   R¹ to R³⁹ independently represent a hydrogen atom, a halogen atom, a nitro group, a cyano group, an amino group, an aryl group with 6 to 20 carbon atoms or a heteroaryl group with 2 to 20 carbon atoms that may be substituted by Z², an alkyl group with 1 to 20 carbon atoms, an alkenyl group with 2 to 20 carbon atoms, an alkynyl group with 2 to 20 carbon atoms that may be substituted by Z³, or a -NHY¹, -NY²Y³, -OY⁴, or -SY⁵ group, and Y¹ to Y⁵ independently represent an aryl group with 6 to 20 carbon atoms or a heteroaryl group with 2 to 20 carbon atoms that may be substituted by Z², or an alkyl group with 1 to 20 carbon atoms, an alkenyl group with 2 to 20 carbon atoms, or an alkynyl group with 2 to 20 carbon atoms that may be substituted by Z³; and
   Z² represents a halogen atom, a nitro group, a cyano group, an amino group, or an alkyl group with 1 to 20 carbon atoms, an alkenyl group with 2 to 20 carbon atoms, or an alkynyl group with 2 to 20 carbon atoms that may be substituted by Z⁴, Z³ represents a halogen atom, a nitro group, a cyano group, an amino group, or an aryl group with 6 to 20 carbon atoms or a heteroaryl group with 2 to 20 carbon atoms that may be substituted by Z⁴, and Z⁴ represents a halogen atom, a nitro group, a cyano group, or an amino group).]
2. The arylamine derivative according to 1, wherein R is a perfluoroalkyl group, and all of R¹ to R³⁹ are hydrogen atoms.
3. The arylamine derivative according to 1 or 2, wherein the cross-linking group is a vinyl group.
4. The arylamine derivative according to any one of 1 to 3, wherein Ar¹ is a 4-vinylphenyl group.
5. A charge-transporting varnish, including: a charge-transporting substance including the arylamine derivative according to any one of 1 to 4; and an organic solvent.
6. A charge-transporting thin film produced using the charge-transporting varnish according to 5.
7. A charge-transporting thin film produced using the charge-transporting varnish according to 5, wherein the thin film has a cross-linking structure formed by a reaction of a cross-linking group of the arylamine derivative represented by formula (1).
8. The charge-transporting thin film according to 6 or 7, that is used for a hole-transporting layer of an organic electroluminescence element.
9. An organic electroluminescence element including the charge-transporting thin film according to 6 or 7.
10. An organic electroluminescence element including an anode and a cathode, and a plurality of functional layers including a hole injection layer, a hole-transporting layer, and a light-emitting layer provided between the anode and the cathode, wherein the hole-transporting layer includes the charge-transporting thin film according to 7.
11. A method for manufacturing an organic electroluminescence element, including: a step of forming a hole injection layer by coating an anode with a varnish for forming a hole injection layer and drying the varnish; and a step of forming a hole-transporting layer by coating the hole injection layer with the charge-transporting varnish according to any one of 1 to 5, and heating the charge-transporting varnish to thermally cross-link the cross-linking group of the arylamine derivative represented by formula (1).
12. The method for manufacturing an organic electroluminescence element according to 11, further including a step of forming a light-emitting layer by coating the hole-transporting layer with a composition for forming the light-emitting layer and drying the composition.
13. A method for manufacturing the arylamine derivative according to 1, wherein a diamine compound represented by formula (5) 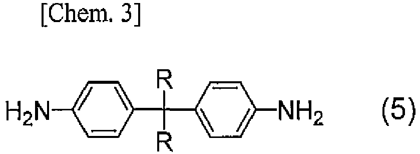
   (wherein, R is the same as above)
   is reacted with an aryl compound represented by formula (6) or formula (7) in the presence of a catalyst
      [Chem. 4]

      Ar¹-X (6) Ar²-X (7)

      (wherein, X represents a halogen atom or a pseudo halogen group, and Ar¹ and Ar² are the same as above), so that a compound represented by formula (8) or formula (9) 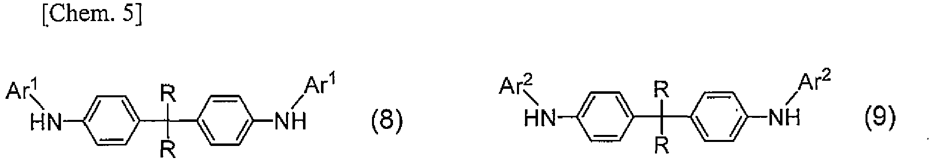
      (wherein, R, Ar¹, and Ar² are the same as above)
      is obtained, and then the compound represented by formula (8) or formula (9) is reacted with the aryl compound represented by formula (7) or formula (6).

### ADVANTAGEOUS EFFECTS OF INVENTION

The arylamine derivative according to the present invention is highly soluble to an organic solvent. By dissolving the arylamine derivative according to the present invention into an organic solvent, a charge-transporting varnish can be easily prepared.

In a charge-transporting thin film formed of the varnish according to the present invention, the cross-linking group in the arylamine derivative is cross-linked and cured, so that the thin film has high solvent resistance. Therefore, this thin film is suitable for the manufacture of a coating type device in which another functional layer is laminated by a coating method. In particular, by using this thin film for the hole-transporting layer of the organic EL element, the light-emitting layer can be easily formed by a coating method.

In addition, the charge-transporting thin film formed of the varnish according to the present invention exhibits the high charge-transporting property; therefore, the thin film is suitably used as a thin film for an electronic device typified by an organic EL element.

Furthermore, the charge-transporting varnish according to the present invention can manufacture the thin film with the excellent charge-transporting property even when various wet processes that can form a film in a large area, such as a spin coating method and a slit coating method, are used. Therefore, the charge-transporting varnish according to the present invention can also be utilized in the recent development in the fields of the organic EL element.

### DESCRIPTION OF EMBODIMENTS

The present invention is described in more detail.

An arylamine derivative according to the present invention is represented by formula (1).

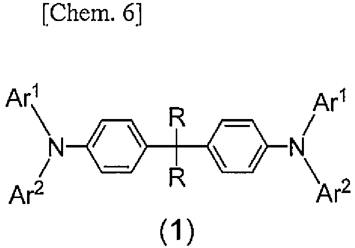

In the formula, R independently represents an alkyl group with 1 to 5 carbon atoms containing a fluorine atom, Ar¹ independently represents an aryl group with 6 to 20 carbon atoms that includes a cross-linking group and may be substituted by Z¹, Ar² independently represents at least one aryl group selected from formulae (2) to (4), and Z¹ represents a halogen atom, a nitro group, a cyano group, an amino group, or an alkyl group with 1 to 20 carbon atoms that may be substituted by Z⁴.

From the viewpoint of synthesis, it is preferable that the groups represented by Ar¹ are the same each other and the groups represented by Ar² are the same each other.

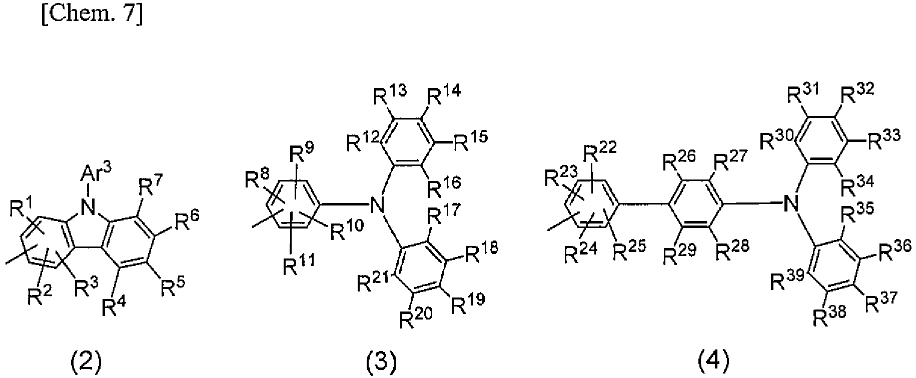

In particular, the groups represented by formulae (2) to (4) are preferably the following groups because the synthesis is easy, for example; however, the groups are not limited to the following groups.

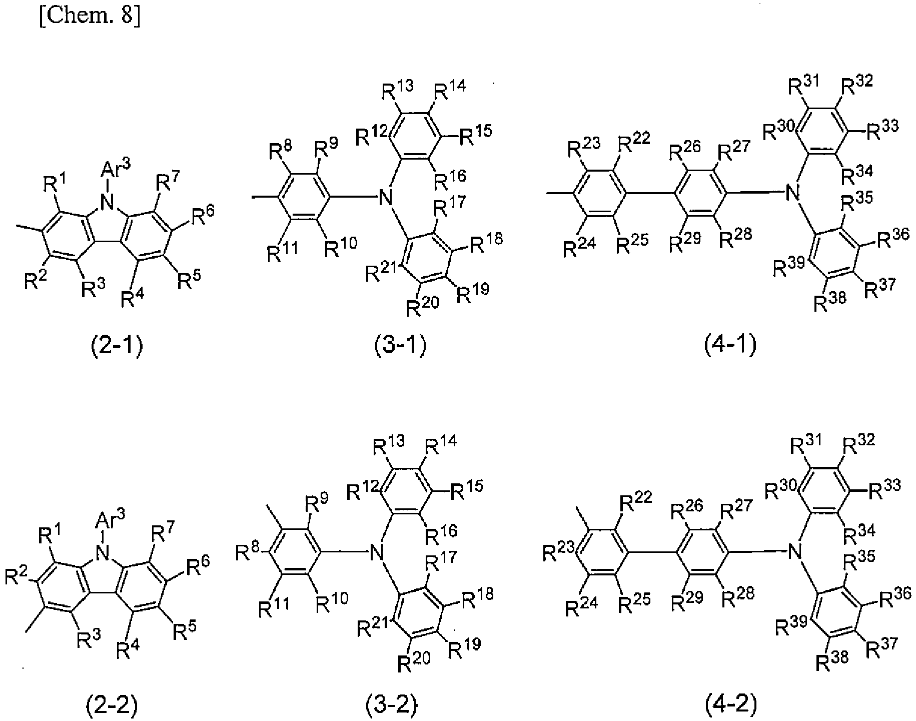

In the formulae, Ar³ represents a hydrogen atom or an aryl group with 6 to 20 carbon atoms that may be substituted by Z²; R¹ to R³⁹ independently represent a hydrogen atom, a halogen atom, a nitro group, a cyano group, an amino group, or an aryl group with 6 to 20 carbon atoms or a heteroaryl group with 2 to 20 carbon atoms that may be substituted by Z², an alkyl group with 1 to 20 carbon atoms, an alkenyl group with 2 to 20 carbon atoms, or an alkynyl group with 2 to 20 carbon atoms that may be substituted by Z³, or a -NHY¹, -NY²Y³, -OY⁴, or -SY⁵ group, and Y¹ to Y⁵ independently represent an aryl group with 6 to 20 carbon atoms or a heteroaryl group with 2 to 20 carbon atoms that may be substituted by Z², or an alkyl group with 1 to 20 carbon atoms, an alkenyl group with 2 to 20 carbon atoms, or an alkynyl group with 2 to 20 carbon atoms that may be substituted by Z³; and Z² represents a halogen atom, a nitro group, a cyano group, an amino group, or an alkyl group with 1 to 20 carbon atoms, an alkenyl group with 2 to 20 carbon atoms, or an alkynyl group with 2 to 20 carbon atoms that may be substituted by Z⁴, Z³ represents a halogen atom, a nitro group, a cyano group, an amino group, or an aryl group with 6 to 20 carbon atoms or a heteroaryl group with 2 to 20 carbon atoms that may be substituted by Z⁴, and Z⁴ represents a halogen atom, a nitro group, a cyano group, or an amino group.

Specific examples of the alkyl group with 1 to 5 carbon atoms containing a fluorine atom include fluoromethyl group, difluoromethyl group, trifluoromethyl group, 2,2,2-trifluoroethyl group, 1,1,2,2,2-pentafluoroethyl group, 3,3,3-trifluoropropyl group, 2,2,3,3,3-pentafluoropropyl group, 1,1,2,2,3,3,3-heptafluoropropyl group, 4,4,4-trifluorobutyl group, 3,3,4,4,4-pentafluorobutyl group, 2,2,3,3,4,4,4-heptafluorobutyl group, and 1,1,2,2,3,3,4,4,4-nonafluorobutyl group.

Specific examples of the aryl group with 6 to 20 carbon atoms include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, and 9-phenanthryl group.

The cross-linking group of the aryl group with 6 to 20 carbon atoms is not limited to a particular group and may be any group that can form a cross-linking structure through mutual reaction. In the present invention, vinyl group, epoxy group, oxetane group, (meth)acryloyl group, (meth)acryloyloxy group, cyclobutenyl group, and the like are preferable. Above all, vinyl group is more preferable. Note that the cross-linking group with a cyclic structure, such as epoxy group and cyclobutenyl group, may be annulated with an aryl group.

Examples of the halogen atom include fluorine atom, chlorine atom, bromine atom, and iodine atom.

The alkyl group with 1 to 20 carbon atoms may be linear, branched or cyclic. Examples thereof include linear or branched alkyl groups with 1 to 20 carbon atoms, such as methyl group, ethyl group, n-propyl group, isopropyl group, n-butyl group, isobutyl group, s-butyl group, t-butyl group, n-pentyl group, n-hexyl group, n-heptyl group, n-octyl group, n-nonyl group, and n-decyl group; and a cyclic alkyl group with 3 to 20 carbon atoms, such as cyclopropyl group, cyclobutyl group, cyclopentyl group, cyclohexyl group, cycloheptyl group, cyclooctyl group, cyclononyl group, cyclodecyl group, bicyclobutyl group, bicyclopentyl group, bicyclohexyl group, bicycloheptyl group, bicyclooctyl group, bicyclononyl group, and bicyclodecyl group.

Specific examples of the heteroaryl group with 2 to 20 carbon atoms include 2-thienyl group, 3-thienyl group, 2-furanyl group, 3-furanyl group, 2-oxazolyl group, 4-oxazolyl group, 5-oxazolyl group, 3-isoxazolyl group, 4-isoxazolyl group, 5-isoxazolyl group, 2-thiazolyl group, 4-thiazolyl group, 5-thiazolyl group, 3-isothiazolyl group, 4-isothiazolyl group, 5-isothiazolyl group, 2-imidazolyl group, 4-imidazolyl group, 2-pyridyl group, 3-pyridyl group, and 4-pyridyl group.

Specific examples of the alkenyl group with 2 to 20 carbon atoms include ethenyl group, n-1-propenyl group, n-2-propenyl group, 1-methylethenyl group, n-1-butenyl group, n-2-butenyl group, n-3-butenyl group, 2-methyl-1-propenyl group, 2-methyl-2-propenyl group, 1-ethylethenyl group, 1-methyl-1-propenyl group, 1-methyl-2-propenyl group, n-1-pentenyl group, n-1-decenyl group, and n-1-eicosenyl group.

Specific examples of the alkynyl group with 2 to 20 carbon atoms include ethynyl group, n-1-propynyl group, n-2-propynyl group, n-1-butynyl group, n-2-butynyl group, n-3-butynyl group, 1-methyl-2-propynyl group, n-1-pentynyl group, n-2-pentynyl group, n-3-pentynyl group, n-4-pentynyl group, 1-methyl-n-butynyl group, 2-methyl-n-butynyl group, 3-methyl-n-butynyl group, 1,1-dimethyl-n-propynyl group, n-1-hexynyl group, n-1-decynyl group, n-1-pentadecynyl group, and n-1-eicosynyl group.

Among these, both R preferably represent perfluoroalkyl group, and more preferably trifluoromethyl group.

Ar¹ preferably represents 2-vinylphenyl group, 3-vinylphenyl group, 4-vinylphenyl group, 2-oxiranylphenyl group, 3-oxiranylphenyl group, 4-oxiranylphenyl group, 2-glycidylphenyl group, 3-glycidylphenyl group, 4-glycidylphenyl group, benzocyclobutenyl group, or the like, and particularly preferably 4-vinylphenyl group.

Ar³ preferably represents hydrogen atom or phenyl group, and more preferably phenyl group.

R¹ to R³⁹ preferably represent hydrogen atoms.

Therefore, Ar² preferably represents a group represented by any of the following formulae (10) to (12), and more preferably a group represented by any of the following formulae (13) to (15).

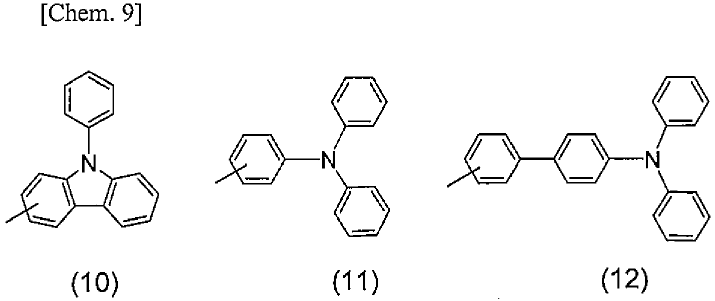

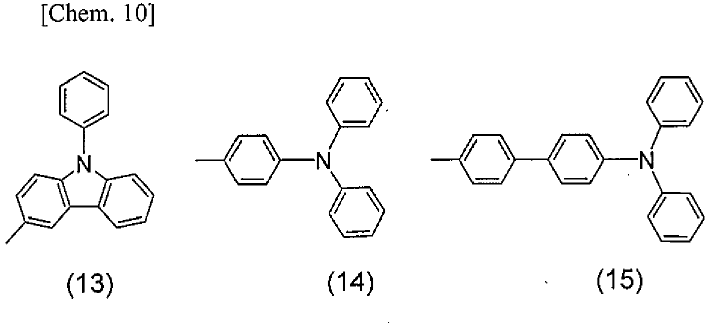

In the arylamine derivative in formula (1) in the present invention, it is preferable that both R represent perfluoroalkyl group, Ar¹ represents 4-vinylphenyl group, and Ar² represents a group represented by any of formulae (10) to (12), it is more preferable that both R represent perfluoroalkyl group, Ar¹ represents 4-vinylphenyl group and Ar² represents a group represented by any of formulae (13) to (15), and it is much more preferable that both R represent trifluoromethyl group, Ar¹ represents 4-vinylphenyl group, and Ar² represents a group represented by any of formulae (13) to (15).

The arylamine derivative represented by the above formula (1) can be obtained as shown in the following scheme: a diamine compound represented by formula (5) and an aryl compound represented by formula (6) or formula (7) are subjected to reaction in the presence of a catalyst so that a compound represented by formula (8) or formula (9) is obtained, and then the compound represented by formula (8) or formula (9) and an aryl compound represented by formula (7) or formula (6) are subjected to reaction. 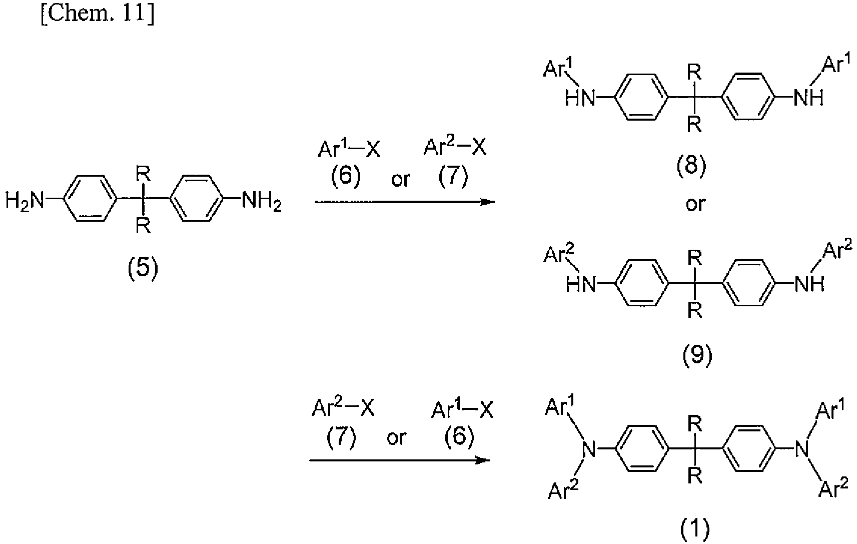 (wherein, X represents a halogen atom or a pseudo halogen group, and R, Ar¹, and Ar² are the same as above.)

The halogen atom may be similar to the above halogen atom.

Examples of the pseudo halogen group include (fluoro)alkylsulfonyloxy groups such as methanesulfonyloxy group, trifluoromethanesulfonyloxy group, and nonafluorobutane sulfonyloxy group, and aromatic sulfonyloxy groups such as benzenesulfonyloxy group and toluenesulfonyloxy group.

In regard to the preparation ratio between the diamine compound represented by formula (5) and the aryl compound represented by formula (6) or formula (7) and the preparation ratio between the diamine compound represented by formula (9) and the aryl compound represented by formula (7) or formula (6), the amount of substance (mol) ratio of the aryl compound to 1 of the diamine compound is preferably approximately 2 to 2.4.

Examples of the catalyst used in the above reaction include copper catalysts such as copper chloride, copper bromide, and copper iodide; and palladium catalysts such as Pd(PPh₃)₄(tetrakis(triphenylphosphine)palladium), Pd(PPh₃)₂Cl₂(bis(triphenylphosphine)dichloropalladium), Pd(dba)₂(bis(dibenzylideneacetone)palladium), Pd₂(dba)₃(tris(dibenzylideneacetone)dipalladium), Pd(P-t-Bu₃)₂(bis(tri(t-butylphosphine))palladium), and Pd(OAc)₂(palladium acetate). These catalysts may be used alone or two or more kinds thereof may be used in combination. These catalysts may be used together with a known appropriate ligand.

Examples of such a ligand include tertiary phosphines such as triphenylphosphine, tri-o-tolylphosphine, diphenylmethylphosphine, phenyldimethylphosphine, trimethylphosphine, triethylphosphine, tributylphosphine, tri-t-butylphosphine, di-t-butyl(phenyl)phosphine, di-t-butyl(4-dimethylaminophenyl)phosphine, 1,2-bis(diphenylphosphino)ethane, 1,3-bis(diphenylphosphino)propane, 1,4-bis(diphenylphosphino)butane, and 1,1'-bis(diphenylphosphino)ferrocene; and tertiary phosphites such as trimethylphosphite, triethylphosphite, and triphenylphosphite.

The used amount of the catalyst may be approximately 0.2 mol, preferably approximately 0.15 mol, per 1 mol of the aryl compound represented by formula (6) or (7).

In the case of using the ligand, the used amount thereof may be 0.1 to 5 equivalents, preferably 1 to 2 equivalents to a metal complex to be used.

In the case where the raw material compound is all solid or from the viewpoint of efficiently obtaining the arylamine derivative as a target, the reactions are performed in a solvent. In the case of using the solvent, the kind of solvent is not limited to a particular kind and may be any solvent that does not adversely affect the reactions. Specific examples thereof include aliphatic hydrocarbons (such as pentane, n-hexane, n-octane, n-decane, and decalin); halogenated aliphatic hydrocarbons (such as chloroform, dichloromethane, dichloroethane, and carbon tetrachloride); aromatic hydrocarbons (such as benzene, nitrobenzene, toluene, o-xylene, m-xylene, p-xylene, and mesitylene); halogenated aromatic hydrocarbons (such as chlorobenzene, bromobenzene, o-dichlorobenzene, m-dichlorobenzene, and p-dichlorobenzene); ethers (such as diethylether, diisopropylether, t-butylmethylether, tetrahydrofuran, dioxane, 1,2-dimethoxyethane, and 1,2-diethoxyethane); ketones (such as acetone, methylethylketone, methylisobutylketone, di-n-butylketone, and cyclohexanone); amides (such as N,N-dimethylformamide and N,N-dimethylacetoamide); lactam and lactones (such as N-methylpyrrolidone and γ-butyrolactone); urines (such as N,N-dimethylimidazolidinone and tetramethylurea); sulfoxides (such as dimethylsulfoxide and sulfolane); and nitriles (such as acetonitrile, propionitrile, and butyronitrile). These solvents may be used alone or two or more kinds thereof may be used in combination.

The reaction temperature may be set as appropriate in the range from the melting point to the boiling point of the solvent to be used, and in particular, is preferably from 0 to 200°C and more preferably 20 to 150°C.

After the reaction, a posterior process is performed in accordance with an established method, and thus, the arylamine derivative as the target can be obtained.

A charge-transporting varnish according to the present invention includes an organic solvent and a charge-transporting substance including the arylamine derivative represented by formula (1).

The organic solvent used for preparing the charge-transporting varnish is not limited to a particular solvent and may be any organic solvent that can dissolve or disperse the arylamine derivative represented by formula (1). Examples thereof include benzene, toluene, o-xylene, m-xylene, p-xylene, N-methylformamide, N,N-dimethylformamide, N,N-diethylformamide, N-methylacetoamide, N,N-dimethylacetoamide, N-methylpyrrolidone, 1,3-dimethyl-2-imidazolidinone, cyclohexanol, ethylene glycol, 1,3-octylene glycol, diethylene glycol, dipropylene glycol, triethylene glycol, tripropylene glycol, 1,3-butane diol, 2,3-butane diol, 1,4-butane diol, propylene glycol, hexylene glycol, butyl cello solve, diethylene glycol diethylether, diethylene glycol dimethylether, diethylene glycol monoethylether acetate, diethylene glycol monobutylether acetate, dipropylene glycol monomethylether, propylene glycol monomethylether, propylene glycol monomethylether acetate, ethylcarbitol, diacetone alcohol, γ-butyrolactone, ethyl lactate, and n-hexylacetate. These may be used alone or two or more kinds thereof may be used in combination.

Among these, aromatic hydrocarbon solvents such as toluene and xylene are preferable.

The solid content concentration of the charge-transporting varnish according to the present invention is set as appropriate in consideration of the viscosity, the surface tension, and the like of the varnish, the thickness of a thin film to be manufactured, and the like. The solid content concentration of the charge-transporting varnish according to the present invention is usually approximately 0.1 to 10.0 wt%, preferably 0.5 to 5.0 wt%, and more preferably 1.0 to 3.0 wt%. Note that the solid content means other components than the organic solvent of the varnish.

When the charge-transporting varnish according to the present invention is prepared, the arylamine derivative and the organic solvent can be mixed in an arbitrary order as long as the solid content is dissolved or dispersed uniformly in the solvent.

Usually, the varnish is prepared in an inert gas atmosphere with normal temperature and normal pressure. However, unless the compound of the varnish is decomposed or the composition thereof is largely changed, the varnish may be prepared under the air atmosphere (in the presence of oxygen) or while heat is applied.

The charge-transporting varnish described above can be used suitably as the varnish for forming a charge-transporting thin film for an organic EL element or the like. Specifically, by coating a base with the charge-transporting varnish according to the present invention and baking the varnish, the charge-transporting thin film can be manufactured.

In particular, the charge-transporting varnish according to the present invention is preferably used as the varnish for forming a hole-transporting layer that is laminated on the hole injection layer.

A coating method of the varnish is not limited to a particular method and may be a dip method, a spin coating method, a transfer printing method, a roll coating method, brush coating, an inkjet method, a spraying method, a slit coating method, or the like. In accordance with a coating method, the viscosity and the surface tension of the varnish are preferably controlled.

In the case of using the varnish according to the present invention, the baking atmosphere is not limited to a particular atmosphere. Not just in the air atmosphere but also in inert gas such as nitrogen or in vacuum, the thin film with a uniform film-formation plane and the high charge transportability can be obtained.

The baking temperature is set as appropriate in the range of approximately 100 to 260°C in consideration of the intended application of the thin film to be obtained, the degree of the charge transportability of the film to be obtained, the kind and the boiling point of the solvent, and the like. In order to sufficiently advance a cross-linking reaction by the cross-linking group of the arylamine derivative represented by formula (1) to form a firm cross-linking structure, the baking temperature is preferably approximately 180 to 250°C, more preferably approximately 190 to 240°C.

When the baking is performed, the temperature may be changed in two stages or more for the purpose of achieving higher uniformity of the film. The heating may be performed using an appropriate instrument such as a hot plate or an oven.

The thickness of the charge-transporting thin film is not limited to a particular thickness, and is preferably 5 to 200 nm when the thin film is used as the hole-transporting layer of the organic EL element. The thickness can be changed by a method of, for example, changing the solid content concentration of the varnish or changing the amount of solution on the substrate in the coating.

The organic EL element according to the present invention includes a pair of electrodes, and the hole-transporting layer or the hole-injecting-and-transporting layer including the charge-transporting thin film according to the present invention between these electrodes. In the case of manufacturing the light-emitting layer that is laminated on the hole-transporting layer by the coating method, the hole-transporting layer preferably has the cross-linking structure in which the arylamine derivative represented by formula (1) is cross-linked.

Typical structures of the organic EL element are the following structures (a) to (f); however, the structures are not limited thereto. Note that in the following structures, an electron-blocking layer or the like may be provided between the light-emitting layer and the anode and a hole-blocking layer or the like may be provided between the light-emitting layer and the cathode as necessary. The hole injection layer, the hole-transporting layer, or the hole-injecting-and-transporting layer may also have a function as the electron-blocking layer or the like, and the electron injection layer, the electron-transporting layer, or the electron-injecting-and-transporting layer may also have a function as the hole-blocking layer or the like.
(a) Anode/hole injection layer/hole-transporting layer/light-emitting layer/electron-transporting layer/electron injection layer/cathode
(b) Anode/hole injection layer/hole-transporting layer/light-emitting layer/electron-injecting-and-transporting layer/cathode
(c) Anode/hole-injecting-and-transporting layer/light-emitting layer/electron-transporting layer/electron injection layer/cathode
(d) Anode/hole-injecting-and-transporting layer/light-emitting layer/electron-injecting-and-transporting layer/cathode
(e) Anode/hole injection layer/hole-transporting layer/light-emitting layer/cathode
(f) Anode/hole-injecting-and-transporting layer/light-emitting layer/cathode

The hole injection layer, the hole-transporting layer, and the hole-injecting-and-transporting layer are layers formed between the light-emitting layer and the anode, and have a function of transporting the holes from the anode to the light-emitting layer. In a case where only one layer of the hole-transporting material is provided between the light-emitting layer and the anode, the layer is the hole-injecting-and-transporting layer. In a case where two or more layers of the hole-transporting material are provided between the light-emitting layer and the anode, the layer closer to the anode is the hole injection layer and the other layers are the hole-transporting layers. In particular, as the hole injection (transporting) layer, not just the thin film that accepts holes from the anode but also the thin film that can easily inject the holes to the hole-transporting (light-emitting) layer is used.

The electron injection layer, the electron-transporting layer, and the electron-injecting-and-transporting layer are layers formed between the light-emitting layer and the cathode, and have a function of transporting the electrons from the cathode to the light-emitting layer. In a case where only one layer of the electron-transporting material is provided between the light-emitting layer and the cathode, the layer is the electron-injecting-and-transporting layer. In a case where two or more layers of the electron-transporting material are provided between the light-emitting layer and the cathode, the layer closer to the cathode is the electron injection layer and the other layers are the electron-transporting layers.

The light-emitting layer is an organic layer with the light-emitting function, and in a case of employing a doping system, the light-emitting layer includes a host material and a dopant material. In this case, the host material has a function of promoting recombination of electrons and holes mainly and confining the excitons in the light-emitting layer, and the dopant material has a function of causing the excitons obtained in the recombination to emit light efficiently. In a case of a phosphorescent element, the host material has a function of confining the excitons that are generated by the dopant mainly in the light-emitting layer.

In the case of manufacturing the organic EL element with the use of the charge-transporting varnish according to the present invention, the material and the manufacturing method as below may be employed but are not limited thereto.

The electrode substrate to be used is preferably cleaned in advance through liquid cleaning with a detergent, alcohol, pure water, or the like. For example, in a case of using an anode substrate, a surface treatment such as a UV ozone process or an oxygen-plasma process is preferably performed just before the substrate is used. However, if the anode material mainly contains an organic substance, the surface treatment may be omitted.

An example of a method for manufacturing the organic EL element including the hole-transporting layer including the thin film obtained from the charge-transporting varnish according to the present invention is as follows.

First, the hole injection layer is formed on the anode substrate, and the charge-transporting varnish according to the present invention is formed by coating on the hole injection layer and baked in accordance with the aforementioned method; thus, the hole-transporting layer is formed.

On the hole-transporting layer, the light-emitting layer, the electron-transporting layer, the electron injection layer, and the cathode are provided in this order. The hole injection layer, the light-emitting layer, the electron-transporting layer, and the electron injection layer may be formed by an evaporation method or a coating method (wet process) in accordance with the characteristic and the like of the material to be used.

Examples of the anode material include a transparent electrode typified by indium tin oxide (ITO) or indium zinc oxide (IZO) and a metal anode including metal typified by aluminum or alloy thereof, for example, and the anode material is preferably the one subjected to a flattening process. A polythiophene derivative or a polyaniline derivative with the high charge transportability can also be used.

Other metals forming the metal anode include scandium, titanium, vanadium, chromium, manganese, iron, cobalt, nickel, copper, zinc, gallium, yttrium, zirconium, niobium, molybdenum, ruthenium, rhodium, palladium, cadmium, indium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and alloy thereof; however, the metal is not limited to those above.

Examples of the material for forming the light-emitting layer include tris(8-quinolinolato)aluminum(III) (Alq₃), bis(8-quinolinolato)zinc(II) (Znq₂), bis(2-methyl-8-quinolinolato)-4-(p-phenylphenolato)aluminum(III) (BAlq), 4,4'-bis(2,2-diphenylvinyl)biphenyl, 9,10-di(naphthalene-2-yl)anthracene, 2-t-butyl-9, 10-di(naphthalene-2-yl)anthracene, 2,7-bis[9,9-di(4-methylphenyl)-fluorene-2-yl]-9,9-di(4-methylphenyl)fluorene, 2-methyl-9,10-bis(naphthalene-2-yl)anthracene, 2-(9,9-spirobifluorene-2-yl)-9,9-spirobifluorene, 2,7-bis(9,9-spirobifluorene-2-yl)-9,9-spirobifluorene, 2-[9,9-di(4-methylphenyl)-fluorene-2-yl]-9,9-di(4-methylphenyl)fluorene, 2,2'-dipyrenyl-9,9-spirobifluorene, 1,3,5-tris(pyrene-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyrene-1-yl)benzene, 1,3-di(pyrene-1-yl)benzene, 6,13-di(biphenyl-4-yl)pentacene, 3,9-di(naphthalene-2-yl)perylene, 3,10-di(naphthalene-2-yl)perylene, tris[4-(pyrenyl)-phenyl]amine, 10,10'-di(biphenyl-4-yl)-9,9'-bianthracene, N,N'-di(naphthalene-1-yl)-N,N'-diphenyl-[1,1':4',1":4",1"'-quaterphenyl]-4,4'''-diamine, 4,4'-di[10-(naphthalene-1-yl)anthracene-9-yl]biphenyl, dibenzo{[f,f']-4,4',7,7'-tetraphenyl}diindeno[1,2,3-cd:1',2',3'-lm]perylene, 1-(7-(9,9'-bianthracene-10-yl)-9,9-dimethyl-9H-fluorene-2-yl)pyrene, 1-(7-(9,9'-bianthracene-10-yl)-9,9-dihexyl-9H-fluorene-2-yl)pyrene, 1,3-bis(carbazole-9-yl)benzene, 1,3,5-tris(carbazole-9-yl)benzene, 4,4',4"-tris(carbazole-9-yl)triphenylamine, 4,4'-bis(carbazole-9-yl)biphenyl (CBP), 4,4'-bis(carbazole-9-yl)-2,2'-dimethylbiphenyl, 2,7-bis(carbazole-9-yl)-9,9-dimethylfluorene, 2,2',7,7'-tetrakis(carbazole-9-yl)-9,9-spirobifluorene, 2,7-bis(carbazole-9-yl)-9,9-di(p-tolyl)fluorene, 9,9-bis[4-(carbazole-9-yl)-phenyl]fluorene, 2,7-bis(carbazole-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazole-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazole, 9-(4-t-butylphenyl)-3,6-ditrityl-9H-carbazole, 9-(4-t-butylphenyl)-3,6-bis(9-(4-methoxyphenyl)-9H-fluorene-9-yl)-9H-carbazole, 2,6-bis(3-(9H-carbazole-9-yl)phenyl)pyridine, triphenyl(4-(9-phenyl-9H-fluorene-9-yl)phenyl)silane, 9,9-dimethyl-N,N-diphenyl-7-(4-(1-phenyl-1H-benzo[d]imidazole-2-yl)phenyl)-9H-fluorene-2-amine, 3,5-bis(3-(9H-carbazole-9-yl)phenyl)pyridine, 9,9-spirobifluorene-2-yl-diphenyl-phosphine oxide, 9,9'-(5-(triphenylsilyl)-1,3-phenylene)bis(9H-carbazole), 3-(2,7-bis(diphenylphosphoryl)-9-phenyl-9H-fluorene-9-yl)-9-phenyl-9H-carbazole, 4,4,8,8,12,12-hexa(p-tolyl)-4H-8H-12H-12C-azadibenzo[cd,mn]pyrene, 4,7-di(9H-carbazole-9-yl)-1,10-phenanthroline, 2,2'-bis(4-(carbazole-9-yl)phenyl)biphenyl, 2,8-bis(diphenylphosphoryl)dibenzo[b,d]thiophene, bis(2-methylphenyl)diphenylsilane, bis[3,5-di(9H-carbazole-9-yl)phenyl]diphenylsilane, 3,6-bis(carbazole-9-yl)-9-(2-ethyl-hexyl)-9H-carbazole, 3-(diphenylphosphoryl)-9-(4-(diphenylphosphoryl)phenyl)-9H-carbazole, and 3,6-bis[(3,5-diphenyl)phenyl]-9-phenylcarbazole. By co-evaporation with a light-emitting dopant, the light-emitting layer may be formed.

Examples of the light-emitting dopant include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino-[9,9a,1gh]coumarin, quinacridone, N,N'-dimethyl-quinacridone, tris(2-phenylpyridine)iridium(III) (Ir(ppy)₃), bis(2-phenylpyridine)(acetylacetonate)iridium(III) (Ir(ppy)₂(acac)), tris[2-(p-tolyl)pyridine]iridium(III) (Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolato]zinc(II), N¹⁰,N¹⁰,N^{10'},N^{10'}-tetra(p-tolyl)-9,9'-bianthracene-10,10'-diamine, N¹⁰,N¹⁰,N^{10'},N^{10'}-tetraphenyl-9,9'-bianthracene-10,10'-diamine, N¹⁰,N^{10'}-diphenyl-N¹⁰,N^{10'}-dinaphthalenyl-9,9'-bianthracene-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)iridium(III), 4,4'-bis[4-(diphenylamino)styryl]biphenyl, bis(2,4-difluorophenylpyridinato)tetrakis(1-pyrazolyl)borate iridium (III), N,N'-bis(naphtharene-2-yl)-N,N'-bis(phenyl)-tris(9,9-dimethylfluorenylene), 2,7-bis{2-[phenyl(m-tolyl)amino]-9,9-dimethyl-fluorene-7-yl)-9,9-dimethyl-fluorene, N-(4-((E)-2-(6((E)-4-(diphenylamino)styryl)naphthalene-2-yl)vinyl)phenyl)-N-phenylbenzeneamine, fac-iridium(III)tris(1-phenyl-3-methylbenzimidazolyn-2-irydene-C,C^{2'}), mer-iridium(III)tris(1-phenyl-3-methylbenzimidazolyn-2-irydene-C,C^{2'}), 2,7-bis[4-(diphenylamino)styryl]-9,9-spirobifluorene, 6-methyl-2-(4-(9-(4-(6-methylbenzo[d]thiazole-2-yl)phenyl)anthracene-10-yl)phenyl)-benzo[d]thiazole, 1,4-di[4-(N,N-diphenyl)amino]styrylbenzene, 1,4-bis(4-(9H-carbazole-9-yl)styryl)benzene, (E)-6-(4-(diphenylamino)styryl)-N,N-diphenylnaphthalene-2-amine, bis(2,4-difluorophenylpyridinato)(5-(pyridine-2-yl)-1H-tetrazolate)iridium(III), bis(3-trifluoromethyl-5-(2-pyridyl)pyrazole)((2,4-difluorobenzyl)diphenylphosphinate)-iridium(III), bis(3-trifluoromethyl-5-(2-pyridyl)pyrazolate)(benzyldiphenylphosphinate)iridium(III), bis(1-(2,4-difluorobenzyl)-3-methylbenzimidazolium)(3-(trifluoromethyl)-5-(2-pyridyl)-1,2,4-triazolate)iridium(III), bis(3-trifluoromethyl-5-(2-pyridyl)pyrazolate)(4',6'-difluorophenylpyridinate)iridium(III), bis(4',6'-difluorophenylpyridinato)(3,5-bis(trifluoromethyl)-2-(2'-pyridyl)pyrolate)-iridium(III), bis(4',6'-difluorophenylpyridinato)(3-(trifluoromethyl)-5-(2-pyridyl)-1,2,4-triazolate)-iridium(III), (Z)-6-mesityl-N-(6-mesitylquinoline-2(1H)-iridene)quinoline-2-amine-BF₂, (E)-2-(2-(4-dimethylamino)styryl)-6-methyl-4H-pyrane-4-iridene)malononitrile, 4-(dicyanomethylene)-2-methyl-6-julolidyl-9-enyl-4H-pyran, 4-(dicyanomethylene)-2-methyl-6-(1,1,7,7-tetramethyljulolidyl-9-enyl)-4H-pyran, 4-(dicyanomethylene)-2-t-butyl-6-(1,1,7,7-tetramethyljulolidine-4-yl-vinyl)-4H-pyran, tris(dibenzoylmethane)phenanthroline europium(III), 5,6,11,12-tetraphenylnaphthacene, bis(2-benzo[b]thiophene-2-yl-pyridine)(acetylacetonate)iridium(III), tris(1-phenylisoquinoline)iridium(III), bis(1-phenylisoquinoline)(acetylacetonate)iridium(III), bis[1-(9,9-dimethyl)-9H-fluorene-2-yl)-isoquinoline](acetylacetonate)iridium(III), bis[2-(9,9-dimethyl-9H-fluorene-2-yl)quinoline](acetylacetonate)iridium(III), tris[4,4'-di-t-butyl-(2,2')-bipyridine]ruthenium(III)·bis(hexafluorophosphate), tris(2-phenylquinoline)iridium(III), bis(2-phenylquinoline)(acetylacetonate)iridium(III), 2,8-di-t-butyl-5,11-bis(4-t-butylphenyl)-6,12-diphenyltetracene, bis(2-phenylbenzothiazolato)(acetylacetonate)iridium(III), 5,10,15,20-tetraphenyltetrabenzoporphyrin platinum, osmium(II)bis(3-trifluoromethyl-5-(2-pyridine)-pyrazolate)dimethylphenylphosphine, osmium(II)bis(3-(trifluoromethyl)-5-(4-t-butylpyridyl)-1,2,4-triazolate)diphenylmethylphosphine, osmium(II)bis(3-(trifluoromethyl)-5-(2-pyridyl)-1,2,4-triazole)dimethylphenylphosphine, osmium(II)bis(3-(trifluoromethyl)-5-(4-t-butylpyridyl)-1,2,4-triazolate)dimethylphenylphosphine, bis[2-(4-n-hexylphenyl)quinoline](acetylacetonate)iridium(III), tris[2-(4-n-hexylphenyl)quinoline]iridium(III), tris[2-phenyl-4-methylquinoline]iridium(III), bis(2-phenylquinoline)(2-(3-methylphenyl)pyridinate)iridium(III), bis(2-(9,9-diethyl-fluorene-2-yl)-1-phenyl-1H-benzo[d]imidazolato)(acetylacetonate)-iridium(III), bis(2-phenylpyridine)(3-(pyridine-2-yl)-2H-chromene-2-onate)iridium(III), bis(2-phenylquinoline)(2,2,6,6-tetramethylheptane-3,5-dionate)iridium(III), bis(phenylisoquinoline)(2,2,6,6-tetramethylheptane-3,5-dionate)iridium(III), iridium(III)bis(4-phenylthieno[3,2-c]pyridinato-N,C^{2'})acetylacetonate, (E)-2-(2-t-butyl-6-(2-(2,6,6-trimethyl-2,4,5,6-tettahydro-1H-pyrrolo[3,2,1-ij]quinoline-8-yl)vinyl)-4H-pyran-4-ylidene)malononitrile, bis(3-trifluoromethyl-5-(1-isoquinolyl)pyrazolate)(methyldiphenylphosphine)ruthenium, bis[(4-n-hexylphenyl)isoquinoline](acetylacetonate)iridium(III), platinum(II)octaethylporphine, bis(2-methyldibenzo[f,h]quinoxaline)(acetylacetonate)iridium(III), and tris[(4-n-hexylphenyl)xoquinoline]irdium(III).

Examples of the material for forming the electron-transporting layer include 8-hydroxyquinolinolate-lithium, 2,2',2"-(1,3,5-benzinetolyl)-tris(1-phenyl-1-H-benzimidazole), 2-(4-biphenyl)5-(4-t-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolate)-4-(phenylphenolato)aluminum, 1,3-bis[2-(2,2'-bipyridine-6-yl)-1,3,4-oxadiazo-5-yl]benzene, 6,6'-bis[5-(biphenyl-4-yl)-1,3,4-oxadiazo-2-yl]-2,2'-bipyridine, 3-(4-biphenyl)-4-phenyl-5-t-butylphenyl-1,2,4-triazole, 4-(naphthalene-1-yl)-3,5-diphenyl-4H-1,2,4-triazole, 2,9-bis(naphthalene-2-yl)-4,7-diphenyl-1,10-phenanthroline, 2,7-bis[2-(2,2'-bipyridine-6-yl)-1,3,4-oxadiazo-5-yl]-9,9-dimethylfluorene, 1,3-bis[2-(4-t-butylphenyl)-1,3,4-oxadiazo-5-yl]benzene, tris(2,4,6-trimethyl-3-(pyridine-3-yl)phenyl)borane, 1-methyl-2-(4-(naphthalene-2-yl)phenyl)-1H-imidazo[4,5f][1,10]phenanthroline, 2-(naphthalene-2-yl)-4,7-diphenyl-1,10-phenanthroline, phenyl-dipyrenylphosphine oxide, 3,3',5,5'-tetra[(m-pyridyl)-phene-3-yl]biphenyl, 1,3,5-tris[(3-pyridyl)-phene-3-yl]benzene, 4,4'-bis(4,6-diphenyl-1,3,5-triazine-2-yl)biphenyl, 1,3-bis[3,5-di(pyridine-3-yl)phenyl]benzene, bis(10-hydroxybenzo[h]quinolinato)beryllium, diphenylbis(4-(pyridine-3-yl)phenyl)silane, and 3,5-di(pyrene-1-yl)pyridine.

Examples of the material for forming the electron injection layer include lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, Li(acac), lithium acetate, and benzoic lithium.

Examples of the cathode material include aluminum, magnesium-silver alloy, aluminum-lithium alloy, lithium, sodium, potassium, and cesium.

Examples of the material for forming the hole injection layer include copper phthalocyanine, titanium phthalocyanine oxide, platinum phthalocyanine, pyradino[2,3-f][1,10]phenanthroline-2,3-dicarbonitrile, N,N,N',N'-tetrakis(4-methoxyphenyl)benzidine, 2,7-bis[N,N-bis(4-methoxy-phenyl)amino]-9,9-spirobifluorene, 2,2'-bis[N,N-bis(4-methoxy-phenyl)amino]-9,9-spirobifluorene, N,N'-diphenyl-N,N'-di[4-(N,N-ditolylamino)phenyl]benzidine, N,N'-diphenyl-N,N'-di[4-(N,N-diphenylamino)phenyl]benzidine, N⁴,N^{4'}-(biphenyl-4,4'-diyl)bis(N⁴,N^{4'},N^{4'}-triphenylbiphenyl-4,4'-diamine)N¹,N^{1'}-(biphenyl-4,4'-diyl)bis(N¹-phenyl-N⁴,N^{4'}-di-m-tolylbenzene-1,4-diamine), and charge-transporting materials according to WO 2004/043117, WO 2004/105446, WO 2005/000832, WO 2005/043962, WO 2005/042621, WO 2005/107335, WO 2006/006459, WO 2006/025342, WO 2006/137473, WO 2007/049631, WO 2007/099808, WO 2008/010474, WO 2008/032617, WO 2008/032616, WO 2008/129947, WO 2009/096352, WO 2010/041701, WO 2010/058777, WO 2010/058776, WO 2013/042623, WO 2013/129249, WO 2014/115865, WO 2014/132917, WO 2014/141998, and WO 2014/132834.

Among these, the aniline derivative and the thiophene derivative disclosed in WO 2005/043962, WO 2013/042623, and WO 2014/141998 are preferable, and the aniline derivative is more preferable, and the aniline derivative represented by the following formulae (H1) to (H2) are much more preferable.

In this case, the molecular weight of the charge-transporting substance that forms the hole injection layer is preferably 200 to 2,000; in consideration of the conductivity, the lower limit of the molecular weight is preferably 300 or more, more preferably 400 or more. In addition, from the viewpoint of making it easier to dissolve into the solvent, the upper limit of the molecular weight is preferably 1,500 or less, more preferably 1,000 or less.

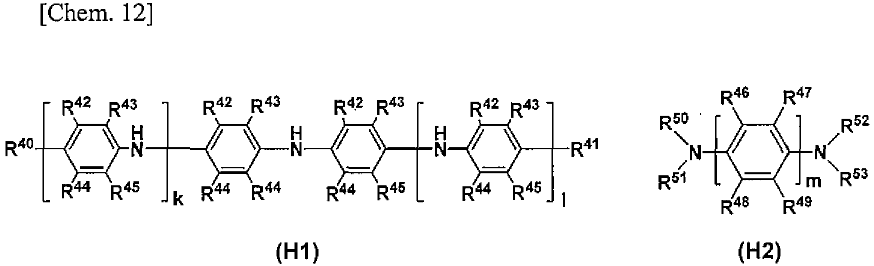

The aniline derivative represented by formula (H1) may be an oxide type aniline derivative with a quinonediimine structure (quinonediimine derivative) that is represented by the following formula in a molecule thereof. As a method of oxidizing the aniline derivative to form the quinonediimine derivative, a method according to WO 2008/010474 or WO 2014/119782 is given.

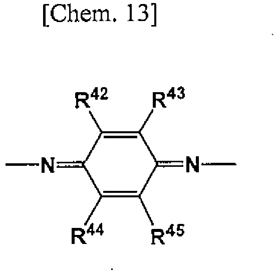

In formula (H1), R⁴⁰ to R⁴⁵ independently represent a hydrogen atom, a halogen atom, a nitro group, a cyano group, an amino group, or an alkyl group with 1 to 20 carbon atoms, an alkenyl group with 2 to 20 carbon atoms, or an alkynyl group with 2 to 20 carbon atoms that may be substituted by Z³, an aryl group with 6 to 20 carbon atoms or a heteroaryl group with 2 to 20 carbon atoms that may be substituted by Z², or -NHY¹, -NY²Y³, -OY⁴, or -SY⁵ group. Y¹ to Y⁵ independently represent an alkyl group with 1 to 20 carbon atoms, an alkenyl group with 2 to 20 carbon atoms, or an alkynyl group with 2 to 20 carbon atoms that may be substituted by Z³, or an aryl group with 6 to 20 carbon atoms or a heteroaryl group with 2 to 20 carbon atoms that may be substituted by Z². Z² and Z³ are the same as those above, and k and 1 independently represent an integer of 1 to 5.

In formula (H2), R⁴⁶ to R⁴⁹ independently represent a hydrogen atom, a halogen atom, a nitro group, a cyano group, a hydroxyl group, a thiol group, a phosphate group, a sulfonic acid group, a carboxyl group, or an alkoxy group with 1 to 20 carbon atoms, a thioalkoxy group with 1 to 20 carbon atoms, an alkyl group with 1 to 20 carbon atoms, an alkenyl group with 2 to 20 carbon atoms, or an alkynyl group with 2 to 20 carbon atoms that may be substituted by Z³, or an aryl group with 6 to 20 carbon atoms, an aralkyl group with 7 to 20 carbon atoms, or an acyl group with 1 to 20 carbon atoms that may be substituted by Z². R⁵⁰ to R⁵³ independently represent a hydrogen atom, a phenyl group, a naphthyl group, a pyridyl group, a pyrimidinyl group, a pyridazinyl group, a pyradinyl group, a furanyl group, a prolyl group, a pyrazolyl group, an imidazolyl group, or a thienyl group (these groups may be substituted by a halogen atom, a nitro group, a cyano group, a hydroxyl group, a thiol group, a phosphate group, a sulfonic acid group, a carboxyl group, an alkoxy group with 1 to 20 carbon atoms, a thioalkoxy group with 1 to 20 carbon atoms, an alkyl group with 1 to 20 carbon atoms, a haloalkyl group with 1 to 20 carbon atoms, an alkenyl group with 2 to 20 carbon atoms, an alkynyl group with 2 to 20 carbon atoms, an aryl group with 6 to 20 carbon atoms, a aralkyl group with 7 to 20 carbon atoms, or an acyl group with 1 to 20 carbon atoms), or a group represented by formula (H3) (note that at least one of R⁵⁰ to R⁵³ represents a hydrogen atom), and m is an integer of 2 to 5. Z² and Z³ are the same as those above.

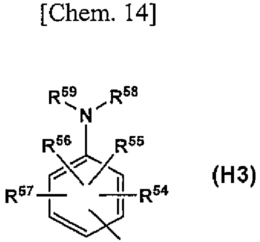

In formula (H3), R⁵⁴ to R⁵⁷ independently represent a hydrogen atom, a halogen atom, a nitro group, a cyano group, a hydroxyl group, a thiol group, a phosphate group, a sulfonic acid group, a carboxyl group, or an alkoxy group with 1 to 20 carbon atoms, a thioalkoxy group with 1 to 20 carbon atoms, an alkyl group with 1 to 20 carbon atoms, an alkenyl group with 2 to 20 carbon atoms, or an alkynyl group with 2 to 20 carbon atoms that may be substituted by Z³, or an aryl group with 6 to 20 carbon atoms, an aralkyl group with 7 to 20 carbon atoms, or an acyl group with 1 to 20 carbon atoms that may be substituted by Z². R⁵⁸ and R⁵⁹ independently represent a phenyl group, a naphthyl group, an anthryl group, a pyridyl group, a pyrimidinyl group, a pyridazinyl group, a pyradinyl group, a furanyl group, a prolyl group, a pyrazolyl group, an imidazolyl group, or a thienyl group (these groups may be connected to form a ring, or may be substituted by a halogen atom, a nitro group, a cyano group, a hydroxyl group, a thiol group, a phosphate group, a sulfonic acid group, a carboxyl group, an alkoxy group with 1 to 20 carbon atoms, a thioalkoxy group with 1 to 20 carbon atoms, an alkyl group with 1 to 20 carbon atoms, a haloalkyl group with 1 to 20 carbon atoms, an alkenyl group with 2 to 20 carbon atoms, an alkynyl group with 2 to 20 carbon atoms, an aryl group with 6 to 20 carbon atoms, an aralkyl group with 7 to 20 carbon atoms, or an acyl group with 1 to 20 carbon atoms). Z² and Z³ are the same as those above.

In the above formulae, specific examples of the halogen atom, the alkyl group with 1 to 20 carbon atoms, the alkenyl group with 2 to 20 carbon atoms, the alkynyl group with 2 to 20 carbon atoms, the aryl group with 6 to 20 carbon atoms, and the heteroaryl group with 2 to 20 carbon atoms are similar to those above.

Specific examples of the aralkyl group with 7 to 20 carbon atoms include benzyl group, phenylethyl group, phenylpropyl group, naphthylmethyl group, naphthylethyl group, and naphthylpropyl group.

One example of the haloalkyl group with 1 to 20 carbon atoms is the alkyl group with 1 to 20 carbon atoms at least one hydrogen atom of which is substituted by a halogen atom. Above all, a fluoroalkyl group is preferable, and a perfluoroalkyl group is more preferable.

Specific examples thereof include fluoromethyl group, difluoromethyl group, trifluoromethyl group, pentafluoroethyl group, 2,2,2-trifluoroethyl group, heptafluoropropyl group, 2,2,3,3,3-pentafluoropropyl group, 2,2,3,3-tetrafluoropropyl group, 2,2,2-trifluoro-1-(trifluoromethyl)ethyl group, nonafluorobutyl group, 4,4,4-trifluorobutyl group, undecafluoropentyl group, 2,2,3,3,4,4,5,5,5-nonafluoropentyl group, 2,2,3,3,4,4,5,5-octafluoropentyl group, tridecafluorohexyl group, 2,2,3,3,4,4,5,5,6,6,6-undecafluorohexyl group, 2,2,3,3,4,4,5,5,6,6-decafluorohexyl group, and 3,3,4,4,5,5,6,6,6-nanofluorohexyl group.

Specific examples of the alkoxy group with 1 to 20 carbon atoms include methoxy group, ethoxy group, n-propoxy group, i-propoxy group, c-propoxy group, n-butoxy group, i-butoxy group, s-butoxy group, t-butoxy group, n-pentoxy group, n-hexoxy group, n-heptyloxy group, n-octyloxy group, n-nonyloxy group, n-decyloxy group, n-undecyloxy group, n-dodecyloxy group, n-tridecyloxy group, n-tetradecyloxy group, n-pentadecyloxy group, n-hexadecyloxy group, n-heptadecyloxy group, n-octadecyloxy group, n-nonadecyloxy group, and n-eicosanyloxy group.

Specific examples of the thioalkoxy (alkylthio) group with 1 to 20 carbon atoms include methylthio group, ethylthio group, n-propylthio group, isopropylthio group, n-butylthio group, isobutylthio group, s-butylthio group, t-butylthio group, n-pentylthio group, n-hexylthio group, n-heptylthio group, n-octylthio group, n-nonylthio group, n-decylthio group, n-undecylthio group, n-dodecylthio group, n-tridecylthio group, n-tetradecylthio group, n-pentadecylthio group, n-hexadecylthio group, n-heptadecylthio group, n-octadecylthio group, n-nonadecylthio group, and n-eicosanylthio group.

Specific examples of the acyl group with 1 to 20 carbon atoms include formyl group, acetyl group, propionyl group, butyryl group, isobutyryl group, valeryl group, isovaleryl group, and benzoyl group.

In formula (H1), R⁴⁰ to R⁴⁵ preferably represent a hydrogen atom, a halogen atom, or an alkyl group with 1 to 20 carbon atoms that may be substituted by Z³, an aryl group with 6 to 20 carbon atoms that may be substituted by Z², -NHY¹, -NY²Y³, -OY⁴, or -SY⁵. In this case, Y¹ to Y⁵ preferably represent an alkyl group with 1 to 10 carbon atoms that may be substituted by Z³ or an aryl group with 6 to 10 carbon atoms that may be substituted by Z², more preferably represent an alkyl group with 1 to 6 carbon atoms that may be substituted by Z³ or a phenyl group that may be substituted by Z², and much more preferably represent an alkyl group with 1 to 6 carbon atoms or a phenyl group.

In particular, it is preferable that R⁴⁰ to R⁴⁵ represent a hydrogen atom, a fluorine atom, a methyl group, a phenyl group, or a diphenylamino group (-NY²Y³ where Y² and Y³ are phenyl groups), and more preferable that R⁴² to R⁴⁵ represent a hydrogen atom and R⁴⁰ and R⁴¹ represent a hydrogen atom or a diphenylamino group at the same time.

Above all, in R⁴⁰ to R⁴⁵ and Y¹ to Y⁵, Z³ preferably represents a halogen atom or an aryl group with 6 to 10 carbon atoms that may be substituted by Z⁴, more preferably a fluorine atom or a phenyl group, and much more preferably, Z³ does not exist (that is, the group is unsubstituted). Moreover, Z² preferably represents a halogen atom or an alkyl group with 1 to 10 carbon atoms that may be substituted by Z⁴, more preferably a fluorine atom or an alkyl group with 1 to 6 carbon atoms, and much more preferably, Z² does not exist (that is, the group is unsubstituted).

In addition, Z⁴ preferably represents a halogen atom, more preferably a fluorine atom, and much more preferably, Z⁴ does not exist (that is, the group is unsubstituted).

In regard to k and 1, from the viewpoint of making it easier to dissolve the aniline derivative represented by formula (H1), preferably k + 1 ≤ 8, more preferably k +1 ≤ 5, is satisfied.

In formula (H2), it is preferable that R⁴⁶ to R⁴⁹ represent a hydrogen atom, a halogen atom, an alkyl group with 1 to 4 carbon atoms, a perfluoroalkyl group with 1 to 4 carbon atoms, or alkoxy group with 1 to 4 carbon atoms, and it is more preferable that R⁴⁶ to R⁴⁹ represent a hydrogen atom.

In order to make it easier to dissolve the aniline derivative represented by formula (H2) in the solvent and to increase the uniformity of a thin film to be obtained, it is preferable that R⁵⁰ and R⁵² both represent a hydrogen atom.

In particular, it is preferable that R⁵⁰ and R⁵² both represent a hydrogen atom and R⁵¹ and R⁵³ independently represent a phenyl group (this phenyl group may be substituted by a halogen atom, a nitro group, a cyano group, a hydroxyl group, a thiol group, a phosphate group, a sulfonic acid group, a carboxyl group, an alkoxy group with 1 to 20 carbon atoms, a thioalkoxy group with 1 to 20 carbon atoms, an alkyl group with 1 to 20 carbon atoms, a haloalkyl group with 1 to 20 carbon atoms, an alkenyl group with 2 to 20 carbon atoms, an alkynyl group with 2 to 20 carbon atoms, an aryl group with 6 to 20 carbon atoms, a aralkyl group with 7 to 20 carbon atoms, or an acyl group with 1 to 20 carbon atoms) or a group represented by the above formula (H3). It is more preferable that R⁵⁰ and R⁵² both represent a hydrogen atom and R⁵¹ and R⁵³ independently represent a phenyl group or a group represented by the following formula (H3') in which R^{58'} and R^{59'} both represent a phenyl group. It is much more preferable that R⁵⁰ and R⁵² both represent a hydrogen atom and R⁵¹ and R⁵³ both represent a phenyl group.

In addition, m is preferably 2 to 4 in consideration of easily obtaining the compound, easily manufacturing the compound, and the cost, and more preferably 2 or 3 in consideration of increasing the solubility to the solvent, and m is optimal at 2 in consideration of the balance among how easily the compound is obtained, how easily the compound is manufactured, the production cost, the solubility to the solvent, the transparency of the thin film to be obtained, and the like.

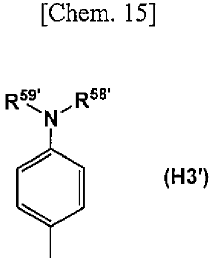

In formula (H3), R⁵⁴ to R⁵⁷ preferably represent a hydrogen atom, a fluorine atom, a sulfonic acid group, an alkyl group with 1 to 8 carbon atoms, a -OY⁴ group, -SiY⁶Y⁷Y⁸ group, and more preferably a hydrogen atom.

The aniline derivatives represented by formulae (H1) and (H2) may be either a commercial product or a derivative manufactured by a known method such as a method disclosed in the above gazette. In either case, it is preferable to use the derivative that is purified by re-crystallization, an evaporation method, or the like before the varnish for forming the hole injection layer is prepared. By using the purified substance, the characteristic of an optical sensor element including the thin film formed of the composition can be increased further. In the case where the purifying is performed by the re-crystallization, the solvent may be, for example, 1,4-dioxane, tetrahydrofuran, or the like.

In the preparation of the varnish for forming the hole injection layer, the aniline derivative represented by formulae (H1) and (H2) may be one kind of compound selected from the compounds represented by formulae (H1) and (H2) (that is, the dispersity of molecular weight distribution is 1) alone or two or more of such compounds may be used in combination.

In particular, from the viewpoint of increasing the transparency of the hole injection layer, the aniline derivative represented by formula (H2) is preferably used. Above all, a benzidine derivative in which m is 2 is more preferably used. Using diphenyl benzidine represented by the following formula (g) is much more preferable.

Specific examples of the aniline derivative that is suitable as the hole injection material are shown below but the aniline derivative is not limited thereto.

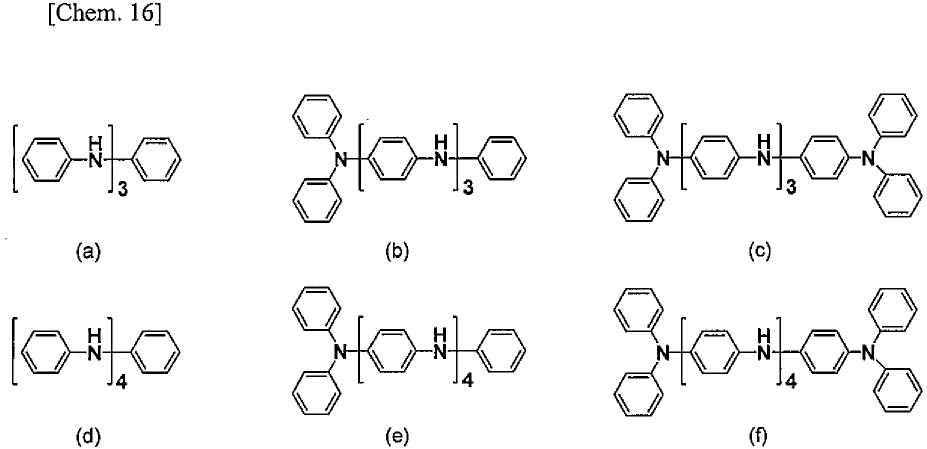

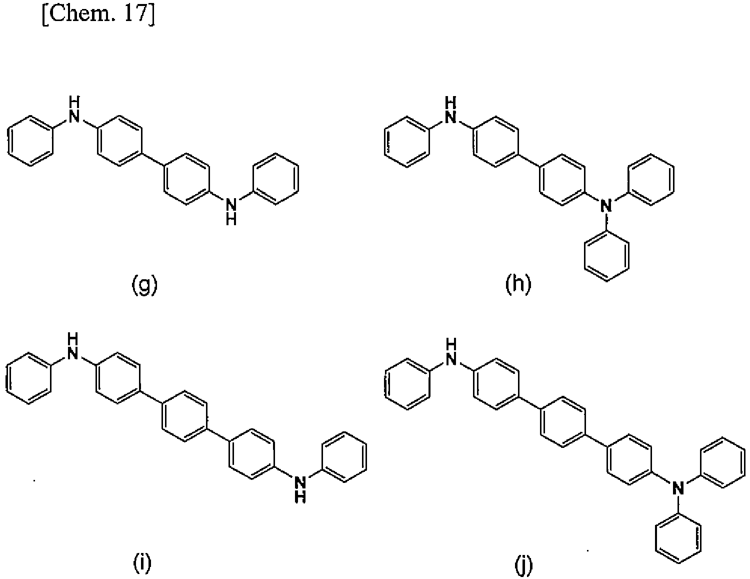

In the preparation of the varnish for forming the hole injection layer, an electron-accepting dopant substance may be added.

The electron-accepting dopant substance is not limited to a particular substance and may be any substance that is dissolved in at least one kind of solvent used in the varnish for forming the hole injection layer.

Specific examples of the electron-accepting dopant substance include inorganic strong acids such as hydrogen chloride, sulfuric acid, nitric acid, and phosphate; Lewis acids such as aluminum chloride(III) (AlCl₃), titanium tetrachloride(IV) (TiCl₄), boron tribromide (BBr₃), boron trifluoride ether complex (BF₃·OEt₂), iron chloride(III) (FeCl₃), copper chloride(II) (CuCl₂), antimony pentachloride(V) (SbCl₅), arsenic pentafluoride(V) (AsFs), phosphorus pentafluoride (PF₅), and tris(4-bromophenyl)aluminum hexachloroantimonato (TBPAH); and organic strong acids such as benzene sulfonic acid, tosic acid, camphorsulfonic acid, hydroxybenzenesulfonic acid, 5-sulfosalicylic acid, dodecylbenzenesulfonic acid, 1,5-naphthalene disulfonic acid, and other naphthalene disulfonic acids, 1,3,5-naphthalene trisulfonic acid, 1,3,6-naphthalene trisulfonic acid, and other naphthalene trisulfonic acids, polystyrene sulfonic acid, 1,4-benzodioxane disulfonic acid compound described in WO 2005/000832, a naphthalene or anthracene sulfonic acid compound described in WO 2006/025342, and an arylsulfonic acid compound such as dinonylnaphthalene sulfonic acid compound described in JP-A 2005-108828; and organic oxidants such as 7,7,8,8-tetracyanoquinodimethane (TCNQ) and 2,3-dichloro-5,6-dicyano-1,4-benzoquinone (DDQ), and iodine, and inorganic oxidants including heteropolyacid such as phosphomolybdic-phosphotungstic acid, phosphotungstic acid or phosphomolybdic acid described in WO 2010/058777. These may be used in combination.

Among these, the arylsulfonic acid compound is preferable, and in particular, the naphthalene or anthracene sulfonic acid compound represented by formula (D1), naphthalene trisulfonic acid such as 1,3,5-naphthalene trisulfonic acid or 1,3,6-naphthalene trisulfonic acid, and polystyrene sulfonic acid are preferable. 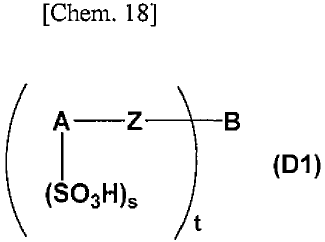 (wherein, Z represents O, A represents a naphthalene ring or an anthracene ring, B represents a divalent to tetravalent perfluorobiphenyl group, s represents the number of sulfonic acid groups that are bound to A, and is an integer satisfying a relation of 1 ≤ s ≤ 4, and t represents the number of bonds between B and Z, and is an integer satisfying 2 to 4.)

Specific examples of the naphthalene or anthracene sulfonic acid compound represented by formula (D1) include the following naphthalene sulfonic acid compound (formula (D2)); however, the compound is not limited the example below.

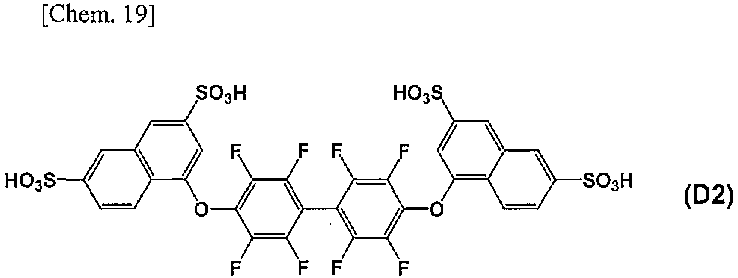

As the organic solvent that is used for preparing the varnish for forming the hole injection layer, a high-solvency solvent capable of dissolving well the material and the electron-accepting dopant substance that is used as necessary can be used. The high-solvency solvent can be used alone or two or more kinds of such solvents can be used in mixture. The used amount thereof may be 5 to 100 wt% for the entire solvent used in the varnish.

Examples of the high-solvency solvent include N-methylformamide, N,N-dimethylformamide, N,N-diethylformamide, N-methylacetoamide, N,N-dimethylacetoamide, N-methylpyrrolidone, and 1,3-dimethyl-2-imidazolidinone.

The charge-transporting substance and the electron-accepting dopant substance are preferably dissolved completely or uniformly dispersed in the organic solvent. In consideration of increasing the repeatability of forming the hole injection layer that achieves the organic EL element with the excellent characteristics, these substances are more preferably dissolved completely in the organic solvent.

The varnish for forming the hole injection layer preferably contains at least one kind of high-viscosity organic solvent that has a viscosity of 10 to 200 mPa·s, particularly 35 to 150 mPa·s at 25°C, and a boiling point of 50 to 300°C, particularly 150 to 250°C at normal pressure.

The high-viscosity organic solvent is not limited to a particular solvent, and examples thereof include cyclohexanol, ethylene glycol, 1,3-octylene glycol, diethylene glycol, dipropylene glycol, triethylene glycol, tripropylene glycol, 1,3-butane diol, 2,3-butane diol, 1,4-butane diol, propylene glycol, and hexylene glycol.

The adding ratio of the high-viscosity organic solvent to the entire solvent used for the varnish for forming the hole injection layer is preferably in the range where the solid does not precipitate. In the range where the solid does not precipitate, the adding ratio is preferably 5 to 80 wt%.

In addition, other solvents that can form the flat film in the thermal treatment can be mixed by 1 to 90 wt%, preferably 1 to 50 wt% for the entire solvent used in the varnish for the purpose of improving the wettability to a coating surface, adjusting the surface tension of the solvent, adjusting the polarity, adjusting the boiling point, and so on.

Examples of such a solvent include butylcellosolve, diethylene glycol diethyl ether, diethylene glycol dimethyl ether, diethylene glycol monoethyl ether acetate, diethylene glycol monobutyl ether acetate, dipropylene glycol monomethyl ether, propylene glycol monomethyl ether, propylene glycol monomethyl ether acetate, ethyl carbitol, diacetone alcohol, γ-butyrolactone, ethyl lactate, and n-hexylacetate. The solvent is, however, not limited thereto.

The solid content concentration of the varnish for forming the hole injection layer is set as appropriate in consideration of the viscosity, the surface tension, and the like of the varnish, the thickness of the thin film to be manufactured, and the like, and is usually approximately 0.1 to 10.0 wt%, preferably 0.5 to 5.0 wt%, and more preferably 1.0 to 3.0 wt%. Note that the solid content refers to other components than the organic solvent.

In addition, the amount of substance (mol) ratio of the electron-accepting dopant substance to the hole injection material is also set as appropriate in consideration of the kind of the charge-transporting material and the hole injection material, for example. Usually, the electron-accepting dopant substance is contained by 0.1 to 10, preferably 0.2 to 5.0, more preferably 0.5 to 3.0 for 1 of the hole injection material.

The viscosity of the varnish for forming the hole injection layer in the present invention is adjusted as appropriate in accordance with the coating method in consideration of the thickness of the thin film to be manufactured, the solid content concentration, and the like, and is usually approximately 0.1 to 50 mPa·s at 25°C.

When the varnish for forming the hole injection layer is prepared, the hole injection material, the electron-accepting dopant substance, and the organic solvent can be mixed at an arbitrary order as long as the solid content is dissolved or dispersed uniformly in the solvent.

In addition, the varnish is prepared usually under an inert gas atmosphere with normal temperature and normal pressure. However, unless the compound in the varnish is decomposed or the composition changes largely, the varnish may be prepared under an air atmosphere (in the presence of oxygen), or while heat is applied.

By coating the anode of the organic EL element with the varnish for forming the hole injection layer and baking the varnish, the hole injection layer according to the present invention can be formed.

The coating method and the baking condition may be similar to those in the formation of the hole-transporting layer described above.

The film thickness is usually 1 to 200 nm, and preferably 3 to 100 nm and more preferably 5 to 30 nm. As a method of changing the film thickness, the solid content concentration of the composition may be changed or the amount of solution in the coating may be changed.

Furthermore, another example of the method of manufacturing the organic EL element including the hole-transporting layer including the thin film formed of the charge-transporting varnish according to the present invention is as below.

By forming the light-emitting layer (hereinafter, light-emitting polymer layer) instead of performing an operation of vacuum evaporation of the light-emitting layer, the electron-transporting layer, and the electron injection layer in the above manufacture of the EL element, the organic EL element including the charge-transporting thin film formed of the charge-transporting varnish according to the present invention can be manufactured.

Specifically, the anode substrate provided with the hole injection layer is coated with the charge-transporting varnish according to the present invention, and the hole-transporting layer is formed by the above method. On the hole-transporting layer, the light-emitting polymer layer is formed and further, the cathode electrode is evaporated; thus, the organic EL element is obtained.

The cathode and anode materials to be used may be similar to those above, and the cleaning process and surface process may also be similar to those above.

As a method for forming the light-emitting polymer layer, solvent is added to a light-emitting polymer material or a light-emitting polymer material including a dopant substance so that the material is dissolved or dispersed uniformly in the solvent. Then, the hole-transporting layer is coated with the mixture and then baked; thus, the light-emitting polymer layer is formed.

Light-emitting polymer materials include polyfluorene derivatives such as poly(9,9-dialkylfluorene) (PDAF), polyphenylene vinylene derivatives such as poly(2-methoxy-5-(2'-ethylhexoxy)-1,4-phenylenevinylene) (MEH-PPV), polythiophene derivatives such as poly(3-alkylthiophene) (PAT), and polyvinylcarbazole (PVCz).

As the solvent, toluene, xylene, chloroform, or the like is given. To dissolve or disperse uniformly, stirring, stirring with heat, ultrasonic dispersion, or the like is performed.

The coating method is not limited to a particular method, and an inkjet method, a spraying method, a dipping method, a spin coating method, a transfer printing method, a roll coating method, brush coating, or the like is given. Note that the coating is preferably performed under inert gas such as nitrogen or argon.

The baking may be performed under inert gas or in vacuum using an oven or a hot plate.

Note that at an arbitrary position between the electrode and any of the above layers, a hole-blocking layer, an electron-blocking layer, or the like may be provided as necessary. For example, as an example of the material for forming the electron-blocking layer, tris(phenylpyrazole)iridium is given.

Since the materials of the anode and the cathode and the materials of the layers formed between these are different depending on whether an element with a bottom emission structure or an element with a top emission structure is manufactured. Thus, the materials are selected as appropriate in consideration of this point.

Usually, in the element with the bottom emission structure, a transparent anode is used on a substrate side and light is extracted from the substrate side; on the other hand, in the element with the top emission structure, a reflection anode made of metal is used and light is extracted from the transparent electrode (cathode) side that is opposite to the substrate. For example, in regard to the anode material, the transparent anode such as ITO is used when the element with the bottom emission structure is manufactured, and the reflection anode such as Al/Nd is used when the element with the top emission structure is manufactured.

In order to prevent the deterioration in characteristic, the organic EL element according to the present invention may be sealed together with a water capturing agent as necessary in accordance with an established method.

### EXAMPLES

With reference to Examples and Comparative Examples, the present invention is hereinafter described more specifically; however, the present invention is not limited to the examples below. Apparatus used here is as shown below.
(1) ¹H-NMR: NMR spectrometer, AVANCE III HD 500 MHz, manufactured by BRUKER
(2) Substrate Cleaning: substrate cleaning device (low-pressure plasma), manufactured by Choshu Industry
(3) Varnish Coating: spin coater MS-A100,
   manufactured by MIKASA CO., LTD
(4) Film Thickness Measurement:
   microfigure measuring instrument, SURFCORDER ET-4000,
   manufactured by Kosaka Laboratory Ltd.
(5) Film Surface Examination:
   confocal laser microscope,
   real-time scanning type laser microscope 1LM21D,
   manufactured by Lasertec Corporation
(6) EL Element Fabrication:
   multifunction vapor deposition system C-E2L1G1-N,
   manufactured by Choshu Industry
(7) Measurement of EL Element Luminance:
   I-V-L measurement system,
   manufactured by Tech World
(8) Measurement of EL Element Lifetime:
   organic EL luminance lifetime evaluation system PEL-105S,
   manufactured by EHC. Co., Ltd.

### [1] Synthesis of arylamine derivative

### [Example 1-1] Synthesis of Arylamine derivative 1

### (1) First step

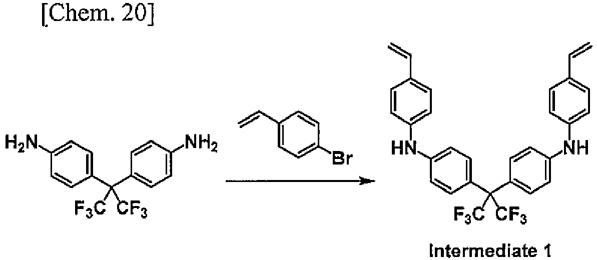

Into a flask, 5.77 g of 4-bromostyrene, 5.01 g of 4,4'-(hexafluoroisopropylidene)dianiline, 0.174 g of Pd(dba)₂, and 4.04 g of t-butoxy sodium were input and the gas in the flask was substituted by nitrogen. Next, 75 mL of toluene and 2.0 mL (concentration: 67 g/L) of toluene solution of di-t-butyl(phenyl)phosphine that was prepared in advance were added thereto, and the mixture was stirred for an hour at 80°C. After the stirring was completed, the reactant mixture was cooled to room temperature, and ethyl acetate and ion exchange water were mixed and the mixture was subjected to a liquid separation process. The obtained organic layer was washed with ion exchange water and saturated saline solution in this order, and dried with magnesium sulfate. This substance was filtered so that the solvent was distilled off under reduced pressure, and separated and purified through a silica-gel column chromatography (developing solvent: n-hexane/ethyl acetate = 85/15 → 80/20), and thus, fractions including the target compound were collected. After the solvent was distilled off under reduced pressure, the mixture was re-crystallized with ethyl acetate/n-hexane. Finally, the crystals were filtered and the obtained filtrate was dried so that an Intermediate 1 was obtained (yield amount: 5.86 g, yield rate: 73%). Shown below are the measurement results of ¹H-NMR.
¹H-NMR (500MHz, DMSO-d₆) δ[ppm]:
8.56(s, 2H), 7.37(d, J = 8.5Hz,4H), 7.20(d, J = 8.5Hz, 4H), 7.12(d, J = 8.5Hz, 4H), 7.11(d, J = 8.5Hz, 4H), 6.65(dd, J = 17.7, 11.0Hz, 2H), 5.65(d, J = 17.7Hz, 2H), 5.10(d, J = 11.0Hz, 2H)

### (2) Second step

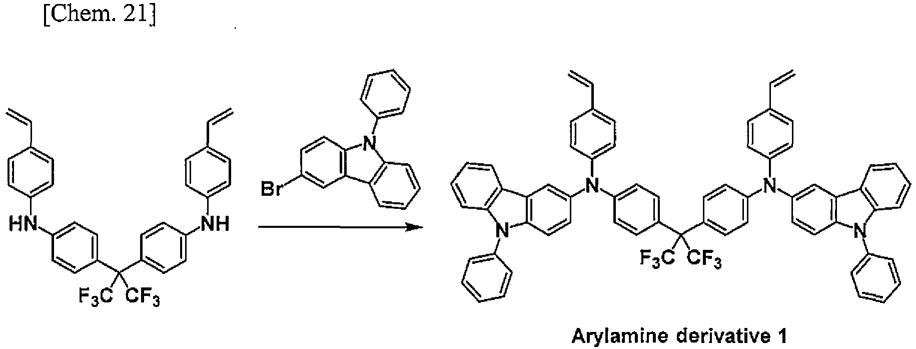

Into a flask, 1.46 g of the Intermediate 1 obtained in the first step, 1.62 g of 3-bromo-9-phenyl-9H-carbazole, 0.148 g of Pd(dba)₂, and 0.673 g of t-butoxy sodium were input, and the gas in the flask was substituted by nitrogen. Next, 20 mL of toluene and 1.4 mL (concentration: 73 g/L) of toluene solution of tri-t-butylphosphine that was prepared in advance were added thereto, and the mixture was stirred for two hours at 80°C. After the stirring was completed, the reactant mixture was cooled to room temperature, and ion exchange water was mixed and the mixture was subjected to a liquid separation process. The obtained organic layer was washed with ion exchange water and saturated saline solution in this order, and dried with magnesium sulfate. This substance was filtered so that the solvent was distilled off under reduced pressure, and separated and purified through a silica-gel column chromatography (developing solvent: n-hexane/chloroform = 67/33 → 0/100), and thus, fractions including the target compound were collected. Then, the solvent was distilled off under reduced pressure. The obtained solid was washed with methanol and then, dried so that an Arylamine derivative 1 was obtained (yield amount: 1.66 g, yield rate: 65%). Shown below are the measurement results of ¹H-NMR.
¹H-NMR (500MHz, THF-d₈) δ[ppm]:
8.06(d, J = 7.9Hz, 2H), 8.03(d, J = 1.8Hz, 2H), 7.59-7.65(m, 8H), 7.47-7.49(m, 2H), 7.31-7.39(m, 10H), 7.16-7.24(m, 8H), 7.12(d, J = 8.9Hz, 4H), 7.02(d, J = 8.9Hz, 4H), 6.66(dd, J = 17.7, 11.0Hz, 2H), 5.64(d, J = 17.7Hz, 2H), 5.10(d, J = 11.0Hz, 2H).

### [Example 1-2] Synthesis of Arylamine derivative 2

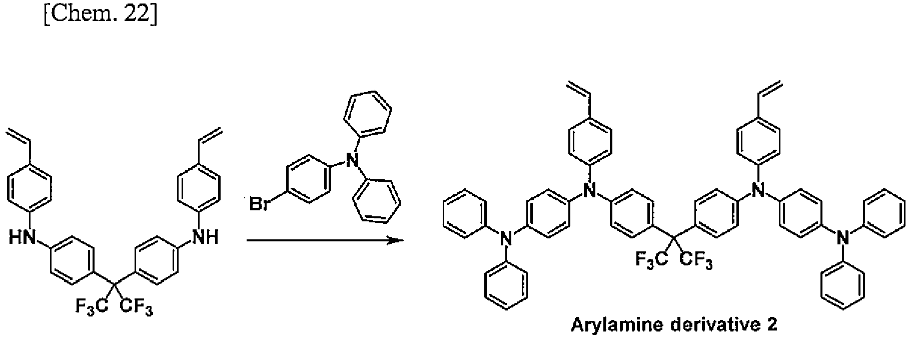

Into a flask, 1.46 g of the Intermediate 1 obtained in the first step, 1.62 g of 4-bromo-N,N-diphenylaniline, 0.146 g of Pd(dba)₂, and 0.676 g of t-butoxy sodium were input, and the gas in the flask was substituted by nitrogen. Next, 20 mL of toluene and 1.7 mL (concentration: 60 g/L) of toluene solution of tri-t-butylphosphine that was prepared in advance were added thereto, and the mixture was stirred for two hours at 80°C. After the stirring was completed, the reactant mixture was cooled to room temperature, and ion exchange water was mixed and the mixture was subjected to a liquid separation process. The obtained organic layer was washed with ion exchange water and saturated saline solution in this order, and dried with magnesium sulfate. This substance was filtered so that the solvent was distilled off under reduced pressure, and separated and purified through a silica-gel column chromatography (developing solvent: n-hexane/chloroform = 67/33 → 60/40), and thus, fractions including the target compound were collected. Then, the solvent was distilled off under reduced pressure. The obtained solid was washed with methanol and then, dried so that an Arylamine derivative 2 was obtained (yield amount: 1.66 g, yield rate: 65%). Shown below are the measurement results of ¹H-NMR.
¹H-NMR (500MHz, THF-d₈) δ[ppm]:
7.35(d, J = 8.5Hz, 4H), 7.21-7.25(m, 12H), 6.96-7.11(m, 28H), 6.67(dd, J = 17.4, 11.0Hz, 2H), 5.66(d, J = 17.4Hz, 2H), 5.13(d, J = 11.0Hz, 2H)

### [Example 1-3] Synthesis of Arylamine derivative 3

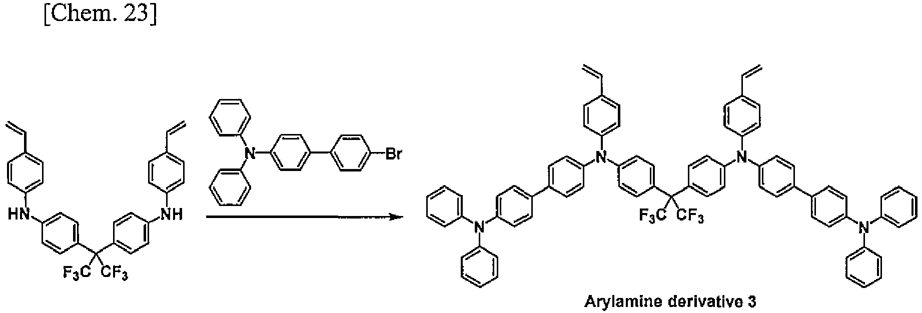

Into a flask, 1.46 g of the Intermediate 1 obtained in the first step, 2.01 g of 4'-bromo-N,N-diphenyl-(1,1'-biphenyl)-4-amine, 0.145 g of Pd(dba)₂, and 0.669 g of t-butoxy sodium were input, and the gas in the flask was substituted by nitrogen. Next, 20 mL of toluene and 1.6 mL (concentration: 63 g/L) of toluene solution of tri-t-butylphosphine that was prepared in advance were added thereto, and the mixture was stirred for two hours at 80°C. After the stirring was completed, the reactant mixture was cooled to room temperature, and ion exchange water was mixed and the mixture was subjected to a liquid separation process. The obtained organic layer was washed with ion exchange water and saturated saline solution in this order, and dried with magnesium sulfate. This substance was filtered so that the solvent was distilled off under reduced pressure, and separated and purified through a silica-gel column chromatography (developing solvent: n-hexane/chloroform = 70/30 → 65/35), and thus, fractions including the target compound were collected. Then, the solvent was distilled off under reduced pressure. The obtained solid was washed with methanol and then, dried so that an Arylamine derivative 3 was obtained (yield amount: 1.15 g, yield rate: 39%). Shown below are the measurement results of ¹H-NMR.
¹H-NMR (500MHz, THF-d₈) δ[ppm]:
7.54(d, J = 8.5Hz, 4H), 7.49(d, J = 8.5Hz, 4H), 7.37(d, J = 8.2Hz, 4H), 7.28(d, J = 8.5Hz, 4H), 7.23(t, J = 7.6Hz, 8H), 7.17(d, J = 8.5Hz, 4H), 7.06-7.11(m, 20H), 6.99(t, J = 7.6Hz, 4H), 6.68(dd, J = 17.4, 11.0Hz, 2H), 5.68(d, J = 17.4Hz, 2H), 5.14(d, J = 11.0Hz, 2H).

### [Example 1-4] Synthesis of Arylamine derivative 4

### (1) Synthesis of 4'-iodine-2,2'-dimethyl-N,N-diphenyl-(1,1'-biphenyl)-4-amine

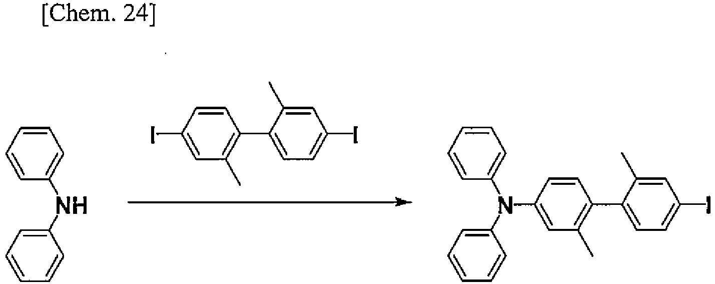

Into a flask, 1.69 g of diphenylamine, 4.34 g of 4,4'-diiodine-2,2'-dimethylbiphenyl, 0.579 g of Pd(PPh₃)₄, and 1.35 g of t-butoxy sodium were input, and the gas in the flask was substituted by nitrogen. Next, 165 mL of toluene was added thereto, and the mixture was stirred for five hours at 110°C. After the stirring was completed, the reactant mixture was cooled to room temperature, and ion exchange water was mixed and the mixture was subjected to a liquid separation process. The obtained organic layer was washed with ion exchange water and saturated saline solution in this order, and dried with magnesium sulfate. This substance was filtered so that the solvent was distilled off under reduced pressure, and separated and purified through a silica-gel column chromatography (developing solvent: n-hexane/chloroform = 100/0 → 85/15), and thus, fractions including the target compound were collected. Then, the solvent was distilled off under reduced pressure. Methanol was added to the concentrate, so that a white solid was precipitated. Then, the solvent was distilled off again under reduced pressure and dried so that 4'-iodine-2,2'-dimethyl-N,N-diphenyl-(1,1'-biphenyl)-4-amine was obtained (yield amount: 1.25 g, yield rate: 26%). Shown below are the measurement results of ¹H-NMR.
¹H-NMR (500MHz, DMSO-d₆) δ[ppm]:
7.69(d, J = 1.2Hz, 1H), 7.57(dd, J = 7.9, 1.2Hz, 1H), 7.30-7.33(m, 4H), 7.03-7.06(m, 6H), 6.97(d, J = 7.9Hz, 1H), 6.93(d, J = 2.1Hz, 1H), 6.90(d, J = 7.9Hz 1H), 6.84(dd, J = 7.9, 2.1Hz, 1H), 2.01(s, 3H), 1.90(s, 3H)

### (2) Synthesis of Arylamine derivative 4

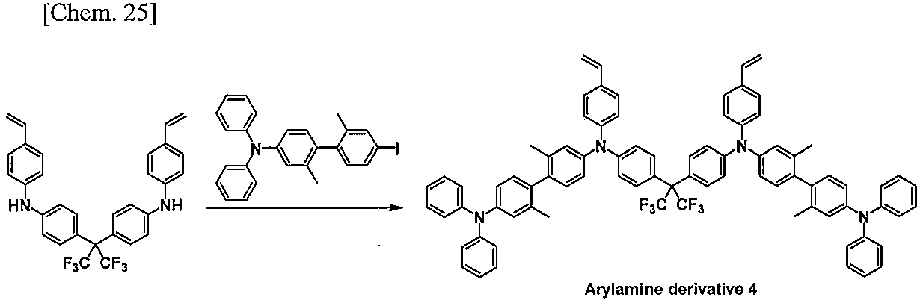

Into a flask, 0.593 g of the Intermediate 1 obtained in the first step in Example 1-1, 1.05 g of 4'-iodine-2,2'-dimethyl-N,N-diphenyl-(1,1'-biphenyl)-4-amine obtained above, 0.130 g of Pd(dba)₂, and 0.425 g of t-butoxy sodium were input, and the gas in the flask was substituted by nitrogen. Next, 10 mL of toluene and 1.5 mL (concentration: 61 g/L) of toluene solution of tri-t-butylphosphine that was prepared in advance were added thereto, and the mixture was stirred for four hours at 80°C. After the stirring was completed, the reactant mixture was cooled to room temperature, and toluene, ethyl acetate, and ion exchange water were mixed and the mixture was subjected to a liquid separation process. The obtained organic layer was washed with ion exchange water and saturated saline solution in this order, and dried with magnesium sulfate. This substance was filtered so that the solvent was distilled off under reduced pressure, and separated and purified through a silica-gel column chromatography (developing solvent: n-hexane/chloroform = 80/20 → 70/30), and thus, fractions including the target compound were collected. Then, the solvent was distilled off under reduced pressure. The obtained solid was washed with methanol and then, dried so that an Arylamine derivative 4 was obtained (yield amount: 0.398 g, yield rate: 29%). Shown below are the measurement results of ¹H-NMR.
¹H-NMR (500MHz, THF-d₈) δ[ppm]:
7.37(d, J = 8.5Hz, 4H), 7.27(d, J = 8.5Hz, 4H), 7.21-7.24(m, 8H), 7.04-7.13(m, 20H), 6.96-7.00(m, 10H), 6.90(dd, J = 8.2, 2.1Hz, 2H), 6.69(dd, J = 17.7, 11.0Hz, 2H), 5.68(d, J = 17.7Hz, 2H), 5.14(d, J = 11.0Hz, 2H), 2.03(s, 6H), 1.99(s, 6H)

### [2] Manufacture of charge-transporting varnish and thin film

### [2-1] Preparation of varnish for forming hole-transporting layer

### [Example 2-1]

To 24 mg of the Arylamine derivative 1 obtained in Example 1-1, 2.0 g of xylene was added and the mixture was stirred at room temperature to dissolve the solid content; thus a solution was obtained. The obtained solution was filtered through a syringe filter with a bore diameter of 0.2 µm, so that a varnish 1 for forming the hole-transporting layer was obtained.

### [Example 2-2]

A varnish 2 for forming the hole-transporting layer was obtained in a manner similar to Example 2-1 except that the Arylamine derivative 2 obtained in Example 1-2 was used.

### [Example 2-3]

A varnish 3 for forming the hole-transporting layer was obtained in a manner similar to Example 2-1 except that the Arylamine derivative 3 obtained in Example 1-3 was used.

### [Example 2-4]

A varnish 4 for forming the hole-transporting layer was obtained in a manner similar to Example 2-1 except that 16 mg of the Arylamine derivative 3 obtained in Example 1-3 and 8 mg of the Arylamine derivative 4 obtained in Example 1-4 were used.

### [Example 2-5]

A varnish 5 for forming the hole-transporting layer was obtained in a manner similar to Example 2-1 except that 12 mg of the Arylamine derivative 3 obtained in Example 1-3 and 12 mg of the Arylamine derivative 4 obtained in Example 1-4 were used.

### [Example 2-6]

A varnish 6 for forming the hole-transporting layer was obtained in a manner similar to Example 2-1 except that 24 mg of the Arylamine derivative 4 obtained in Example 1-4 was used.

### [2-2] Preparation of varnish for forming hole injection layer

### [Reference Example 1] Varnish for forming hole injection layer

To a mixture including 58.9 mg (0.086 mmol) of oligo aniline compound represented by formula (c) and 160.9 mg (0.161 mmol) of arylsulfonic acid compound represented by formula (D2), 5.00 g of DMAc was added and the mixture was stirred at 40°C so that the solid content was dissolved. To the obtained mixture, 5.0 g of CHN was added and stirred, so that a dark green solution was obtained. This dark green solution was filtered through a syringe filter with a bore diameter of 0.2 µm, so that the varnish 1 for forming the hole injection layer was obtained.

The oligo aniline compound represented by formula (c) was synthesized in accordance with the method described in WO 2014/141998.

### [3] Production of charge-transporting thin film and solvent resistance test

### [Examples 3-1 to 3-3]

An ITO substrate was coated with the varnish 1 for forming the hole injection layer obtained in Reference Example 1 using a spin coater, dried at 80°C for one minute, and further baked for 15 minutes at 230°C under an air atmosphere. Thus, a uniform thin film with a thickness of 30 nm was formed on the ITO substrate. The ITO substrate with the thin film was coated with the varnishes 1 to 3 for forming the hole-transporting layer obtained in Examples 2-1 to 2-3 using a spin coater, and the varnishes were baked under each condition according to Table 1 under an N₂ atmosphere; thus, a uniform thin film with a thickness of 20 nm was formed.

On the formed thin film, 0.5 ml of toluene was applied and left for one minute, and then the toluene was removed by spin coating. In addition, drying with heat was performed at 100°C for one minute. Then, the film thickness was measured. From the film thickness before and after the toluene stripping, the residual film ratio was calculated. The results are shown in Table 1.

**[Table 1]**

| | Residual film ratio (%) | | |
|---|---|---|---|
| Baking condition | 120°C, 10 min | 120°C, 30 min | 200°C, 30 min |
| Example 3-1 | 0 | 0 | 98 |
| Example 3-2 | 0 | 0 | 99 |
| Example 3-3 | 0 | 0 | 96 |

As shown in Table 1, the cross-linking reaction of the arylamine derivative does not progress at 120°C; therefore, the manufactured film is all dissolved in toluene. In the baking at 200°C for 30 minutes, however, the cross-linking reaction of the arylamine derivative progresses; therefore, the manufactured thin film is hardly dissolved in toluene.

Thus, it is understood that the charge-transporting thin film including the cross-linked product of the arylamine derivative according to the present invention can also be used for the element in which the upper light-emitting layer is formed by coating.

### [4] Fabrication and evaluation of organic EL element

### [Example 4-1]

The ITO substrate was coated with the varnish 1 for forming the hole injection layer obtained in Reference Example 1 using a spin coater, dried at 80°C for one minute, and baked for 15 minutes at 230°C under an air atmosphere. Thus, a uniform thin film (hole injection layer) with a thickness of 100 nm was formed on the ITO substrate. The ITO substrate was a glass substrate with a size of 25 mm × 25 mm × 0.7 t on which indium tin oxide (ITO) was patterned with a thickness of 150 nm. Before the use, impurities on the surface were removed by an O₂ plasma cleaning device (150 W, 30 seconds).

Next, the ITO substrate provided with the thin film was coated with the varnish 1 for forming the hole-transporting layer obtained in Example 2-1 by a spin coater. Then, baking was performed at 200°C for 30 minutes, so that the uniform thin film (hole-transporting layer) with a thickness of 20 nm was formed on the hole injection layer.

On the manufactured hole-transporting layer, CBP and Ir(PPy)₃ were co-evaporated using an evaporation device (vacuum degree 1.0 × 10⁻⁵ Pa). In the co-evaporation, the evaporation rate was controlled so that the concentration of Ir(PPy)₃ became 6%, and a layer with a thickness of 40 nm was laminated. Next, thin films of Alq₃, lithium fluoride, and aluminum were sequentially laminated, so that the organic EL element was obtained. In this case, the evaporation rate was 0.2 nm/s for Alq₃ and aluminum, and 0.02 nm/s for lithium fluoride, and the film thicknesses were 20 nm, 0.5 nm, and 80 nm, respectively.

In order to prevent the deterioration in characteristic due to oxygen, water, and the like in air, the organic EL element was sealed by sealing substrates and then, the characteristics thereof were evaluated. The sealing was performed in accordance with the following procedure. In a nitrogen atmosphere with an oxygen concentration of 2 ppm or less and a dew point of -85°C or less, the organic EL element was set between the sealing substrates and the sealing substrates were attached together with an adhesive material (XNR5516Z-B1, manufactured by Nagase ChemteX Corporation). In this process, a water capturing agent (HD-071010W-40, manufactured by DYNIC CORPORATION) was set within the sealing substrates together with the organic EL element. After the attached sealing substrates were irradiated with UV light (wavelength: 365 nm, radiation: 6,000 mJ/cm²), the substrates were annealed at 80°C for one hour, so that the adhesive material was cured.

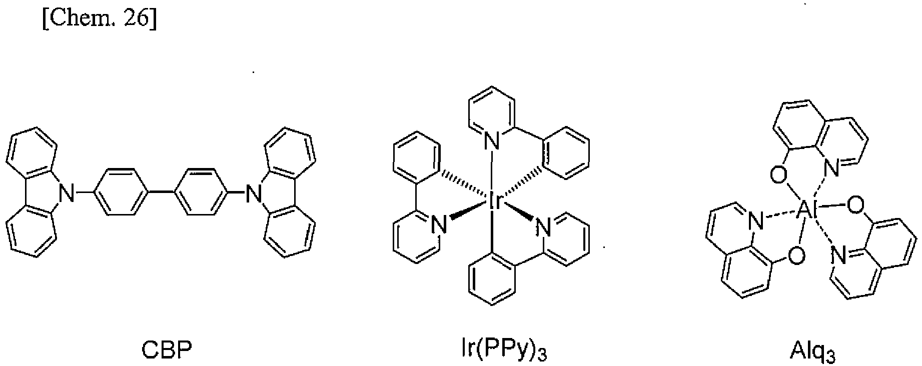

### [Examples 4-2, 4-3]

Organic EL elements were obtained in a manner similar to Example 4-1 except that the varnishes 2 and 3 for forming the hole-transporting layer obtained in Examples 2-2 and 2-3, respectively, were used.

### [Examples 4-4 to 4-6]

Organic EL elements were obtained in a manner similar to Example 4-1 except that the varnishes 4 to 6 for forming the hole-transporting layer obtained in Examples 2-4 to 2-6, respectively were used.

### [Comparative Example 4-1]

An organic EL element was obtained in a manner similar to Example 4-1 except that CBP and Ir(PPy)₃ were directly co-evaporated on the hole injection layer.

In regard to the element manufactured in Examples 4-1 to 4-6 and Comparative Example 4-1, the driving voltage and the current efficiency in a case of driving the element at a luminance of 500 cd/m² and the half-life of the luminance (time required for the initial luminance 500 cd/m² to become a half, not measured in Example 3-2) were measured. The results are shown in Table 2.

**[Table 2]**

| | Driving voltage (V) | Current efficiency (cd/A) | Half-life period (hour) |
|---|---|---|---|
| Example 4-1 | 8.86 | 17.86 | 505 |
| Example 4-2 | 8.54 | 15.03 | - |
| Example 4-3 | 8.84 | 15.11 | 578 |
| Example 4-4 | 9.76 | 13.77 | 650 |
| Example 4-5 | 10.08 | 13.64 | - |
| Example 4-6 | 11.29 | 13.77 | - |
| Comparative Example 4-1 | 8.04 | 8.49 | 500 |

As shown in Table 2, it is understood that the EL element including the charge-transporting thin film according to the present invention as the hole-transporting layer is excellent in current efficiency and lifetime characteristic.

## Claims

1. An arylamine derivative represented by formula (1): 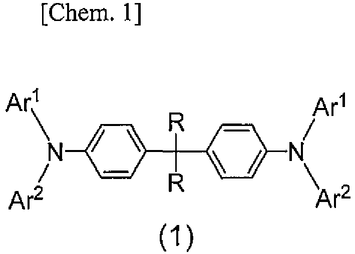 [wherein, R independently represents an alkyl group with 1 to 5 carbon atoms containing a fluorine atom, Ar¹ independently represents an aryl group with 6 to 20 carbon atoms that includes a cross-linking group and may be substituted by Z¹, Ar² independently represents at least one aryl group selected from formulae (2) to (4), and Z¹ represents a halogen atom, a nitro group, a cyano group, an amino group, or an alkyl group with 1 to 20 carbon atoms that may be substituted by Z⁴, 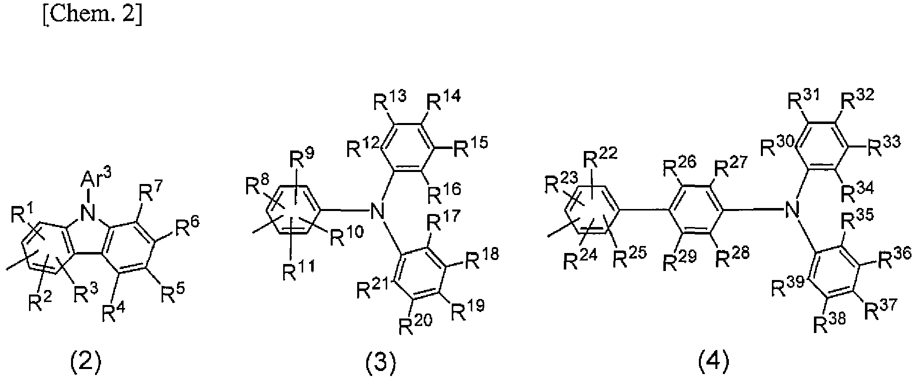
(wherein, Ar³ represents a hydrogen atom or an aryl group with 6 to 20 carbon atoms that may be substituted by Z²;
R¹ to R³⁹ independently represent a hydrogen atom, a halogen atom, a nitro group, a cyano group, an amino group, an aryl group with 6 to 20 carbon atoms or a heteroaryl group with 2 to 20 carbon atoms that may be substituted by Z², an alkyl group with 1 to 20 carbon atoms, an alkenyl group with 2 to 20 carbon atoms, an alkynyl group with 2 to 20 carbon atoms that may be substituted by Z³, or a -NHY¹, -NY²Y³, -OY⁴, or -SY⁵ group, and Y¹ to Y⁵ independently represent an aryl group with 6 to 20 carbon atoms or a heteroaryl group with 2 to 20 carbon atoms that may be substituted by Z², or an alkyl group with 1 to 20 carbon atoms, an alkenyl group with 2 to 20 carbon atoms, or an alkynyl group with 2 to 20 carbon atoms that may be substituted by Z³; and
Z² represents a halogen atom, a nitro group, a cyano group, an amino group, or an alkyl group with 1 to 20 carbon atoms, an alkenyl group with 2 to 20 carbon atoms, or an alkynyl group with 2 to 20 carbon atoms that may be substituted by Z⁴, Z³ represents a halogen atom, a nitro group, a cyano group, an amino group, or an aryl group with 6 to 20 carbon atoms or a heteroaryl group with 2 to 20 carbon atoms that may be substituted by Z⁴, and Z⁴ represents a halogen atom, a nitro group, a cyano group, or an amino group).]

2. The arylamine derivative according to claim 1, wherein R is a perfluoroalkyl group, and all of R¹ to R³⁹ are hydrogen atoms.

3. The arylamine derivative according to claim 1 or 2, wherein the cross-linking group is a vinyl group.

4. The arylamine derivative according to any one of claims 1 to 3, wherein Ar¹ is a 4-vinylphenyl group.

5. A charge-transporting varnish, comprising:
a charge-transporting substance including the arylamine derivative according to any one of claims 1 to 4; and
an organic solvent.

6. A charge-transporting thin film produced using the charge-transporting varnish according to claim 5.

7. A charge-transporting thin film produced using the charge-transporting varnish according to claim 5, wherein the thin film has a cross-linking structure formed by a reaction of a cross-linking group of the arylamine derivative represented by formula (1).

8. The charge-transporting thin film according to claim 6 or 7, that is used for a hole-transporting layer of an organic electroluminescence element.

9. An organic electroluminescence element comprising the charge-transporting thin film according to claim 6 or 7.

10. An organic electroluminescence element comprising an anode and a cathode, and a plurality of functional layers including a hole injection layer, a hole-transporting layer, and a light-emitting layer provided between the anode and the cathode, wherein the hole-transporting layer includes the charge-transporting thin film according to claim 7.

11. A method for manufacturing an organic electroluminescence element, comprising:
a step of forming a hole injection layer by coating an anode with a varnish for forming a hole injection layer and drying the varnish; and
a step of forming a hole-transporting layer by coating the hole injection layer with the charge-transporting varnish according to any one of claims 1 to 5, and heating the charge-transporting varnish to thermally cross-link the cross-linking group of the arylamine derivative represented by formula (1).

12. The method for manufacturing an organic electroluminescence element according to claim 11, further comprising a step of forming a light-emitting layer by coating the hole-transporting layer with a composition for forming the light-emitting layer and drying the composition.

13. A method for manufacturing the arylamine derivative according to claim 1, wherein a diamine compound represented by formula (5) 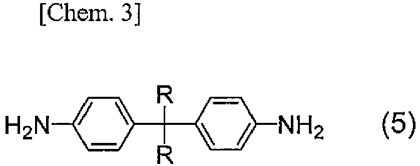
(wherein, R is the same as above)
is reacted with an aryl compound represented by formula (6) or formula (7) in the presence of a catalyst
[Chem. 4]
Ar¹-X (6)
Ar²-X (7)
(wherein, X represents a halogen atom or a pseudo halogen group, and Ar¹ and Ar² are the same as above), so that a compound represented by formula (8) or formula (9) 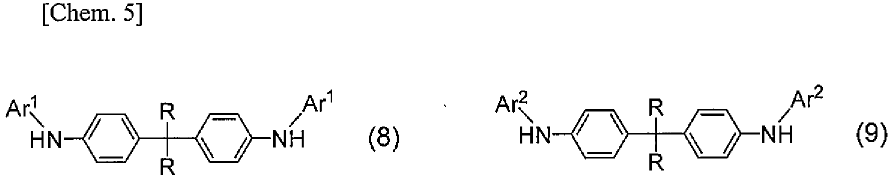
(wherein, R, Ar¹, and Ar² are the same as above).
is obtained, and then the compound represented by formula (8) or formula (9) is reacted with the aryl compound represented by formula (7) or formula (6).
